# EUROPEAN PATENT APPLICATION

(11) **EP 4 846 969 A2**
(43) Date of publication of application: **02.12.2026**
(21) Application number: 26212523.0
(22) Date of filing: 18.07.2019
(51) Int. Cl.: A61B 18/26

(54) **METHOD AND APPARATUS FOR LASER LITHOTRIPSY**

(30) Priority: 18.07.2018 US 201862700019 P
(62) Document number(s) of the earlier application(s) in accordance with Art. 76 EPC: 25195134.9 / 4 620 416; 19846635.1 / 3 806 771
(71) Applicant: IPG Photonics Corporation, Marlborough, MA 01752 (US)
(72) Inventor: Altshuler, Gregory B., Marlborough, MA, 01752 (US); Kovalenko, Anastasia, Marlborough, MA, 01752 (US); Vinnichenko, Victoria, Marlborough, MA, 01752 (US); YAROSLAVSKY, Ilya, Marlborough / MA, 01752 (US)
(74) Representative: Kobiako von Gamm, Iouri

(57) **Abstract**

The present invention provides a laser system for treating calculi in a human or animal body, comprising:
a diode-pumped laser configured to emit radiation in a wavelength range of 1.85 µm to 2.2 µm; and
a driver configured to provide modulated current to one or more pump diodes of the diode-pumped laser and coupled to a controller configured to output a control signal containing information on a desired laser pulse shape such that the diode-pumped laser emits a sequence of laser pulses each having the desired laser pulse shape, wherein:
the desired laser pulse shape is formed with an initial pulse section and a subsequent pulse section,
the subsequent pulse section has a higher power than a power of the initial pulse section, and the power of the initial pulse section is monotonically increased from a minimal power level to the power of the subsequent pulse section,
the minimal power level of the initial pulse section varying in a range of 0 W to 200 W,
a duration of the initial pulse section varying in a range of 0.1 ms to 10 ms,
an energy of the initial pulse section varying in a range of 10 % to 70 % of a total energy of the laser pulse,
the power of the subsequent pulse section varying in a range of 400 W to 20 kW, and
a duration of the subsequent pulse section varying in a range of 0.5 ms to 20 ms.

## Description

### BACKROUND OF THE INVENTION

### 1. Field of the Invention

The disclosure is generally directed to methods and laser systems for treating calculi in human or animal body with controlling the temporal structure of laser power to increase the speed of stone treatment in accordance with various surgical techniques. In particular, the invention relates a method and laser system for treating a calculus with laser pulses having an improved temporal structure by means of modulation of the pulse energy, peak power, and repetition rate as well as pulse shape.

### 2. Discussion of Related Art

Pulsed laser sources may be used in lithotripsy procedures for eliminating calculi in humans and animals. A calculus (also referred to herein as a stone) is a concretion of material that may form in an organ or duct of the body. Urinary calculi include kidney stones (which may also be called renal calculi or nephroliths) as well as bladder stones (which may also be called vesical calculi or cystoliths) and may have any one of a variety of compositions, including mixed compositions. Principal compositions often include calcium oxalate, calcium phosphate, magnesium ammonium phosphate, diammonium calcium phosphate, magnesium phosphate, cysteine, uric acid or urate, and xanthine. Calculi of the gallbladder and bile ducts are called gallstones and are primarily developed from bile salts and cholesterol derivatives. Calculi may also form in other areas of the body, including the nasal passages, gastrointestinal tract, salivary glands, tonsils, and veins.

Laser lithotripsy is the use of a laser to induce damage of stone through various mechanisms. A fiber optic that travels along a longitudinal axis of a rigid or flexible or ridged endoscope typically transmits the laser beam for stone fragmentation. Stone can be split into particles with dimensions between 1 and 3 to 4 mm (fragmentation), which can subsequently be removed through the working channel of a rigid instrument using a basket or a similar implement. Alternatively, the stone can be split into smaller particles (<1 mm in size) in a process referred to as dusting. A subclass of dusting known as fine dusting (particles < 0.25 to 0.5 mm, depending on the stone composition and dust particle's shape) results in fragments sufficiently small to be removed by urine flow or by standard irrigation flow supplied by a water/saline bag hung at about 40 cm height. Vaporization down to molecular level is also possible. Efficiency of ablation depends on conditions at the treatment site and can be substantially lower for large stones or when treating multiple stones in a single surgical procedure. This problem is more typical for treatment of large kidney stones and multiple kidney stones with a flexible ureteroscope when the goal is to complete stone dusting in contact and non-contact modes during surgery.

Another problem is movement of the stone during treatment due to retropulsion. Retropulsion is caused by the following phenomena. Optical energy absorbed by water in the gap between the fiber and the stone produces a water pressure wave, which pushes the stone in a direction away from the tip of the fiber. When laser energy is absorbed by the stone and ablation of the stone takes place, a recoil momentum of ablation also causes the stone to be displaced away from the fiber. Retropulsion prolongs the operation time and makes it difficult for the surgeon to complete fragmentation or dusting of the stones and achieve a residual stone particle-free outcome. Increasing the laser average power and time of treatment to compensate for a lower ablation rate can be limited by increasing the risk of soft tissue damage.

Several surgical techniques for performing laser lithotripsy are known and based on a mutual position and relative displacement of, for example, fiber tip and stone to be treated. These techniques generally fall into one of the following categories: contact fragmentation, quasi-contact scanning (dancing), and non-contact (popcorn) techniques. When using the fragmentation technique, the fiber tip is placed in contact with the stone center and laser power is delivered to the stone until stone macro-cracking and fragmentation occurs. In fragmentation technique laser power is applied in one small area for a relatively long period of time. Such a mode of operation results in relatively deep drilled holes and thermo-mechanical stress that induces stone macro-cracking.

In scanning technique, the fiber is in quasi contact with the stone (distance 0 -1 mm) and continuously moves across the stone surface. Every pass across the stone surface results in removing (ablating) a thin layer of stone. This technique is preferable for ablating stones into small fragments (dusting). Non-contact technique is used for treatment of small stone fragments (typically below 3 mm in size) if retropulsion does not allow for the possibility to operate in contact or quasi-contact modes. In non-contact technique, the fiber is positioned at a fixed location close to the location of targeted stone fragments and the laser is fired in a non-contact fashion. The water vaporization and bubble implosions result in water streaming, which causes the small stone fragments to move. When such fragments enter the effective range of the distal end of the surgical fiber, further fragmentation/dusting occurs, resulting in even smaller stone fragments.

The ablation rate is determined by the ablation mechanism, which depends on the chemical composition and structure of the stone, the operational parameters of the laser (e.g., wavelength, radiation energy, peak power, , pulse width, and repetition frequency), on the thickness of the interlayer between the end of the fiber supplying radiation and the surface of the stone, and on optical properties (transparency) of the substance in this interlayer. Various physical processes and mechanisms of ablation accompany the above-mentioned surgical techniques of stone destruction and include photomechanical, photothermal, and/or photochemical mechanisms.

The photomechanical mechanism which is typical for laser pulses that are shorter than several microseconds asserts that ablation is initiated when the laser-induced tensile stress exceeds the ultimate tensile strength of the target. Key factors in this mechanism include the mechanical properties of the target material and the laser induced stress. Transient tensile stress within a solid material lead to the formation of microscopic cracks and other defects, and if the stress exceeds the effective strength of the material, fracture and material ejection can occur (a process called spallation). In addition, transient tensile stresses in liquids can rupture the medium, causing a phenomenon known as cavitation. Cavitation involves the growth and collapse of cavities within liquid, and can cause damage to surrounding solid material. Laser energy can also lead to plasma formation on the matter. Plasma formation is achieved through fast matter ionization with optical breakdown, which is a non-linear effect produced when laser radiation is strongly absorbed by irradiated matter and/or with high power density on the target. In liquid treatment environments, laser-induced bubble formation in the liquid is caused by direct or plasma-mediated absorption. This absorption creates a growing vapor bubble. Although the growing vapor bubble may not reach the stone, energy from the pulse is still absorbed by the liquid. Thus, bipolar pressure pulses (i.e., shockwave) are formed in front of the growing bubble, which induces cracking of the stone. A cavitation process with the bubble further contributes to shockwave growth. Thus, focusing laser energy on a target material damages the material by the sequence of plasma formation with optical breakdown, shockwave generation, and induction of negative pressure during bubble cavitation.

The photothermal mechanism is typical for a laser having a pulse longer than about a dozen microseconds. Submillisecond and millisecond lasers having a wavelength close to the water absorption peak at 1940 nm (in the range 1.85 to 2.1 µm) are preferable for the lithotripsy with silica fibers used for energy delivery. Water is the single most important initial chromophore in stone facilitating conversion of laser energy into thermal and thermal-mechanical energy that is responsible for fracturing stones in the infrared spectrum transmitted through silica fiber.

The photothermal mechanism can be performed in two modes. The first mode is stone dusting, and it occurs due to elevated pressure caused by the expansion or evaporating water initially confined in stones (about 10% of stone weight/weight) between the stone microcrystals, within stone pores, fissures, initial micro-cracks, and other micro-spaces. Heating and subsequent boiling is caused by selective absorption of water surrounded by mineral and organic components of the stone that by themselves have low absorption of light at about 2 µm wavelength. This mechanism leads to separation of fragments ranging in size from a characteristic dimension of a basic microcrystal (up to several hundred microns, predominantly from submicron's to several dozen microns) or their clusters/domains with characteristic dimensions up to 0.5 mm (fine dusting) or 1 mm (dusting). The second mode is stone fragmentation (to fragments > 1 mm) and it is predominantly due to thermal stresses in the bulk of the stone around the area heated by the laser and dominates dusting when laser power is applied to one point on the stone (stone drilling). The first mode is practically always present regardless of the surgical technique, but it dominates in scanning and pop-corning surgical techniques. The second mode is relevant for very high pulse energies and for fragmentation technique.

The photochemical mechanism of ablation is based on absorption of high energy photons which leads to a direct dissociation of molecular bonds in the material. The photochemical mechanism may play a role to increase absorption of the stone due to carbonization of organic molecules within the stone structure or thermal chemical reaction in the mineral matrix. The surgical procedures, such as fine dusting, dusting and fragmentation, ablation efficiency, and the retropulsion effect can by controlled by laser parameters such as pulsewidth, energy per pulse, pulse repetition rate, and average power. In addition, the method of energy delivery, e.g., fiber parameters such as core diameter, numerical aperture, distal tip conditions, and the distance between the distal end of the fiber and the stone, also plays an important role. Finally, the drilling, scanning or non-contact application all also contribute to the outcome of laser-stone interaction.

Several types of lasers may be used in laser lithotripsy procedures and are selected based on different criteria. For example, the holmium:YAG (Ho:YAG) flash lamp pumped lasers lithotripter is typically operated at high pulse energies (0.1 - 6J), but is limited to low pulse rates (5 -100 Hz) during lithotripsy procedures. The pulse shape and control of other characteristics of temporal nature is very limited for this laser due to flash lamp pumping.

Other lasers in addition Ho:YAG used in laser lithotripsy and operating with wavelengths close to the water absorption peak at about 1.94 µm include, but not limited to: diode pumped thulium Tm:YAG laser, and diode pumped Tm fiber laser (TFL). In particular, diode pumped Tm fiber laser has many advantageous characteristics. These lasers can operate at a broad pulse energy range (0.001 - 20 J), and at low and high pulse rates (1 - 1000000 Hz). Comparison studies TFL and Ho:YAG configurations have been performed. (see, for example Blackmon et al., Journal of Biomedical Optics, 16(7): 071403, July 2011). In addition, the effect of laser pulse operational parameters, such as power settings or on reducing retropulsion, have been studied (see, for example, White, et al., Journal of Endourology, 12(2):183-186, March 2009 and Andreeva V, et al. World journal of urology. 2019 May 4:1-7.). The emission line of the TFL can be adjusted in the range between 1.85 and 2.2 µm and made to be very close to an absorption peak of water around 1.94 µm (1.94 µm for water at about 20 - 30°C and 1.908 µm for water at about 90-100°C). TFL ablation thresholds for different stone compositions are much lower (about 5 times) than those of Ho:YAG lithotripsy systems, which translates into a lower pulse energy for the same ablation rate or equivalent pulse energy but more efficient stone ablation than Ho:YAG systems.

The beam profile of fiber lasers, including TFL, is thus more uniform and symmetrical than the multimodal beam of the Ho:YAG laser or other solid state lasers, which cannot be coupled into small-core fibers due to inevitable damage to the fiber. For instance, single-mode (SM) thulium fiber lasers are capable of focusing the laser beam down to about 25 microns. The smaller fiber diameters provided by the TFL enable focusing of higher power densities than in the case of holmium lasers, which also decreases retropulsion. The smaller fiber diameter increases the radiant exposure or irradiance on the stone surface, which means that lower laser pulse energies may be used during laser ablation processes. The smaller fiber diameters are important when the fiber is used in a flexible ureteroscope having a small working channel, both for improving the irrigation flow and for not compromising the deflection angle of the ureteroscope. The improved spatial beam profile provided by thulium fiber lasers reduces laser-induced damage to the proximal fiber tip surface, which translates into a longer operating life than a holmium-based system. Use of smaller diameter fibers (less than 150 microns (µm), preferably between 50 and 125 µm) can increase the efficiency of fine dusting through focusing laser energy between clusters (domains) of microcrystals and into cracks and fissures on the stone surface. Holmium laser systems also generate heat, which contributes to beam misalignment, which in turn has the potential to cause fiber damage.

As a group, diode-pumped fiber lasers are capable of operating at a greater variety of laser operating parameters than conventional flash lamp-pumped, solid-state lasers such as the Ho:YAG system. For instance, single-laser-head Ho:YAG systems are typically limited to pulse rates of <30 Hz due to the potential of overheating and causing thermal damage to the laser rod. The white light from the flash lamp is mostly wasted in the form of heat, with only a small portion contributing to actual operation of the laser, and these systems typically have a wall-plug efficiency of <1-2%. Large and bulky cooling devices (e.g., water cooling devices) are therefore required for these systems to dissipate this heat and prevent damage to the laser rod. In contrast, diode-pumped fiber laser systems have a wall-plug efficiency of about 10 -50%, which means that a much smaller cooling device (e.g., air-cooled) may be used. The increased wall-plug efficiency also enables fiber-based systems to operate at a higher average power than holmium-based systems, but still be capable of using a 110-120 V electrical outlet.

Other advantages of diode pumped Tm fiber lasers include their ability to generate different temporal structures including broad ranges of pulse shapes, which can also be implemented with other diode pumped lasers emitting in the range 1.85 - 2.2 µm. For example, Tm:YAG laser with diode laser pumping has this capacity. The above discussed advantages of diode pumped solid state and fiber lasers are incorporated in the present invention, as will be discussed in the present application.

Problems associated with the development of laser lithotripsy include reducing the time of the operation, which is associated with the rate of ablation of stones, the path (speed) of the movement of the fragments (e.g., reducing retropulsion), as well as the products of destruction. This problem can be significantly ameliorated by optimizing the temporal structure of the laser emission; this capability is provided by diode pumped fiber or solid state lasers.

All lasers used for laser lithotripsy are operated with a preset energy per pulse (E=0.025 - 6 J), repetition rate (*v*=1 - 2500 Hz), pulsewidth and average power P=E x *v* (2 - 120 W). Laser parameters (E, *v*, and P) are pre-selected to achieve desired results: fragmentation, dusting or popcorning and to provide safety margin that should minimize the risk of organ wall perforation or water overheating and thermal burning of mucosa. However, stones, as mentioned above, have different compositions, shapes and sizes. While the fixed set of the mentioned parameters can be can effectively breaks one specific stone, it may be ineffective for another one.

For achieving minimal retropulsion with high ablation efficiency, it was proposed to extend pulsewidth (long pulse mode) or use special dual pulse regime. US Patent No. 5,321,715 proposes a method of irradiating a target (a stone as an example), in which the space between a target and fiber end normally occupied by liquid medium absorbing laser radiation is made transparent in two steps: (1) generating a first laser pulse having sufficient energy to form a vapor bubble in the liquid medium at the delivery end of the fiber, and (2) generating a second laser pulse at a predetermined time interval after the first pulse, where the predetermined time interval is selected to allow the vapor bubble to expand an amount sufficient to displace a substantial portion of the liquid medium from the space between the delivery end of the target so that the second laser pulse may be delivered to the target through the vapor bubble, thereby minimizing the laser radiation absorbed by the liquid medium and maximizing the laser radiation reaching the target. Thus, the first pulse creates a vapor channel between the fiber end and the target (Moses effect) and the second pulse propagates to the target with minimal losses and interaction with liquid. However, the solution taught by this patent brought only partial success.

A need therefore exists for a shaped pulsed laser lithotripsy performed by a laser system operative to modulate the pulse energy, peak power, pulse repetition frequency and pulse shape to provide optimum conditions for laser-stone interaction leading to decreased treatment time and cost.

### SUMMARY

This goal is satisfied by the inventive laser system and method for treating calculi in a human or animal body. Generally, there are two aspects of the inventive concept of providing high efficiency ablation rates while minimizing undesirable effects of retropulsion: 1. Modulation or periodic variation of the pulse energy, peak power, and pulse frequency, and 2. Formation and maintenance of optimal pulse shapes. Each of the aspects is discussed in view of a laser system for treating calculi and a method of treating the calculi. The aspects are complementary to one another and the below-discussed structural features of one of the aspects can be used in the other aspect, as will be readily apparent to one of ordinary skill in the laser and urological arts.

The methods of respective aspects provide multi-component sized particles in the range of smaller than 1mm, preferably smaller than 0.5 mm, and most preferably below 0.25 mm by using a diode pumped fiber laser or diode pumped solid state laser which operate in a 1.85 - 2.2 µm wavelength range at a specific range of laser power density. Particles smaller than 500 µm and preferably smaller than 250 µm can be easily removed by irrigation flow through a ureteroscope configured with a pressure that is safe for the kidney (<40 cm water column height).

The method in accordance with the first aspect relates to the modulation of the pulse energy E and/or pulse peak power Pp and is referred to as amplitude modulation (AM), as well as the modulation of pulse frequency (i.e., pulse repetition rate) *v* defined as frequency modulation (FM). Finally, the method relates to simultaneous amplitude and frequency modulation - amplitude frequency modulation (AFM).

In particular, the method for treating calculi in a human or animal body in accordance with the first aspect relates to the AM and includes emitting the sequence of laser pulses from a laser at a constant pulse repetition frequency (PRF), and with a periodically varying peak power or pulse energy or peak power and pulse energy with an amplitude modulation period Na ≥ 2.

The method dealing with AFM emitting a sequence of laser pulses provides for periodically varying at least one of pulse peak power or pulse energy or pulse peak power and energy with a modulation period Na equal to the number of pulses in an amplitude periodic group of pulses, and periodically varying PRF with a frequency modulation period Np equal to the number of pulses in a frequency periodic group of pulses.

Both of the above methods of the first aspect are implemented with the modulation period Na ranging from 2 to 1000 laser pulses, preferably from 2 to 100 laser pulses, most preferably from 2 to 10 laser pulses, the modulation period Np of the PRF varies from 2 to 1000 laser pulses, preferably from 2 to 100 laser pulses, most preferably from 2 to 10 laser pulses. In both methods, the laser pulses are emitted in a wavelength range of 1.85 to 2.2 µm and preferably in a 1.91 to 1.96 µm wavelength range.

The second aspect of the invention related to the formation of optimal pulse shapes formed in accordance with the following two embodiments.

In particular, in one embodiment, the method for treating calculi in a human or animal body includes outputting a control signal containing information on a desired laser pulse shape. In response to the control signal a sequence of laser pulses is emitted such that each laser pulse with the desired laser shape which is formed with a first and second sub-pulses spaced temporarily from one another. The energy of the first sub-pulse varies in a range of 0.02 to 0.15 J (preferably 0.05 to 0.1 J) and its peak power ranges from 50 to 500 W (preferably 100 to 300 W). The sub-pulses are temporally by an interval varying in a range 50 to 900 us with a 100 to 500 us range being preferable. The energy of the second sub-pulse exceeds that of the first sub-pulse and varies in a range of 0.1 to 10 J, whereas its peak power exceeds the peak power of the first sub-pulse and varying in a range of 300 to 20000 W.

The other embodiment relates the method for treating calculi in a human or animal body and includes outputting a control signal containing information on a desired laser pulse shape. In response to the control signal, a sequence of laser pulses emitted such that the desired laser pulse shape has an initial and subsequent sections, with the subsequent session having a higher power level than the initial section. The power of the initial section being monotonically increased from a minimal power level to the power level of the subsequent session with the minimal power level varying between 0 and 200 W. The duration of the initial section varies in a 0.1 to 10 ms range, while the energy of the initial pulse section constitutes from 10 to 70% of a total energy of pulse. The subsequent section has the power varying between 400 and 20000 W and the duration of the subsequent section varying in a 0.5 to 20 ms range.

In accordance with the above-mentioned one aspect of the invention, the inventive laser system for treating calculi provides the cluster-type mechanism of laser ablation of multi-component solids by configuring a pulse repetitive laser. In particular, the laser is configured with modulated laser radiation and controlled shape of the laser pulse for simultaneously (i) controlling the size of the ablation products, (ii) enhancing the ablation rate, and (iii) decreasing stone retropulsion. Modulated laser emission and controlled shape of the laser pulse can be used for enhancing the ablation rate and decreasing retropulsion in the fragmentation mode as well.

In accordance with one feature of this aspect, the inventive laser system is configured with a laser emitting a sequence of laser pulses and operable in an AM regime (AMR). In this regime, the laser emits laser pulses at a constant pulse repetition frequency (PRF) and periodically varying at least one of peak power and pulse energy with an amplitude modulation period Na equal to the number of laser pulses in an amplitude periodic group of pulses.

In accordance with another feature, the inventive laser system includes the laser operable in an amplitude-frequency modulation regime (AFMR) This regime is characterized by the laser emitting a sequence of laser pulses with a periodically varied pulse peak power or pulse or both with a modulation period Na equal to the number of pulses in an amplitude periodic group of pulses. The AMPR regime further provides for the sequence of emitted pulses with a periodically varied PRF with a frequency modulation period Np equal to the number of pulses in a frequency periodic group of pulse.

The type of lasers utilized in either of the above-featured inventive laser systems preferably includes a diode-pumped solid state laser and a diode-pumped fiber laser such as pumped solid state laser. In particular, the diode pumped lasers are Tm:YAG, Tm:YLF, Tm:YAP, Tm:LuAG, Tm:LuLF, Tm:LuAP and Tm fiber laser. However, a flash lamp-pumped solid state laser such as Ho:YAG can be employed in the inventive laser system. Furthermore, the use of a direct diode laser is not excluded from the scope of the present invention.

The modulation period Na of the periodically varied peak power, pulse energy or both ranges from 2 to 100 laser pulses and is applicable to both featured laser systems. The modulation period of at least one of peak power or pulse energy or both in the AMR ranges from 2 to 1000 laser pulses. The period Np of the periodically varied PF in the AFMR varies from 2 to 1000 laser pulses.

The lasers utilized in both featured configurations of the inventive laser system emit laser pulses in a wavelength range of 1.85 to 2.2 µm and preferably in a 1.908 to 1.96 µm wavelength range. The operation at these wavelengths allows predominant absorption of laser radiation by water which is highly beneficial to any of the above-mentioned surgical techniques.

The above mentioned lasers used in both featured configurations can operate in the free running mode and Q-switch with a structural difference therebetween being a type of modulator. The modulator utilized in the free-running regime includes a diode laser, whereas, the Q-switch mode is associated with an acoustic-optical or electro-optical modulator (AOM and EOM, respectively).

In the free running mode, any of the disclosed lasers output the sequence of laser pulses with the PRF ranging between 2 and 5000 Hz. Each laser pulse has the following characteristics: a laser pulse energy in a 0.001 J - 10 J range, a laser pulse peak power in a 100 - 20000 W, preferable 250-3000W range, and a laser pulse duration in a 25 µs - 50 ms range, with a 100 µs - 15 ms range being preferable.

The lasers operating in Q-switch mode with modulated quality of the resonator output the laser pulses which are characterized by the following pulse characteristics: the energy varying between 0.1 and 10 mJ, peak power ranging between 200 and 1000000 W, and PF ranging between 500 and 500000 Hz.

The inventive system of both featured laser configurations includes a controller outputting a control signal containing information on the desired peak power or pulse energy in the AMR or desired peak power or pulse energy, and desired PRF in the AFMR. The signal is coupled into a driver of any of the above-mentioned modulators.

In accordance with the other aspect, the inventive laser system is configured with a controller outputting a control signal which contains information on a desired laser pulse shape. The laser is operatively coupled to the controller such that in response to the control signal each laser pulse has a desired laser pulse shape. The shaped laser pulse is formed with a first and second sub-pulses spaced temporarily from one another. The energy of the first sub-pulse varies in a range of 0.02 to 0.15 J (preferably 0.05 to 0.1 J) and has a peak power of the first sub-pulse varying in a range of 50 to 500 W (preferably 100 to 250 W). The interval between the first and the second sub-pulses varies in a range 50 to 900 us with a 100 to 500 us range being preferable. The second sub-pulse has the energy exceeding the energy of the first sub-pulse and varying in a range of 0.1 to 10 J, and a peak power exceeding the peak power of the first sub-pulse and varying in a range of 300 to 20000 W.

Still another configuration of the inventive laser system in accordance with this aspect is structured with a controller outputting a control signal containing information on the desired laser pulse shape. A laser is operatively coupled to the controller and emits a sequence of laser pulses in response to the control signal so that each laser pulse is formed with the desired laser pulse shape. The shaped laser pulse is formed with an initial and subsequent sections with the subsequent section having a power higher than that of the initial section.

The initial section has the power which monotonically increases from a minimal power level varying between 0 and 200 W to the power level of the subsequent section varying between 400 and 20000 W. The duration of the initial section varies in a 0.1 to 10 ms range with the of the initial section being 10-70% of a total energy of pulse, whereas the duration of the subsequent section varying in a 0.5 to 20 ms range. The power of the initial section increases in accordance with one of linear, polynomial, and exponential functions.

Still other aspects, structural details, and advantages of the invention are discussed in detail below in the specific description. Moreover, it is to be understood that both the foregoing information and the following detailed description are merely illustrative examples of various aspects and embodiments, and are intended to provide an overview or framework for understanding the nature and character of the claimed aspects and embodiments. As can be readily understood, all of the above and below disclosed structural particularities of the invention may be combined in any reasonable combination readily understood by one of ordinary skill in laser and urological fields.

### BRIEF DESCRIPTION OF THE DRAWINGS

The aspects of the invention are further discussed below with reference to the accompanying figures, which are not intended to be drawn to scale. The figures are included to provide an illustration and a further understanding of the various structural features, and are incorporated in and constitute a part of this specification, but are not intended as a definition of the limits of any particular embodiment. The drawings, together with the remainder of the specification, serve to explain principles and operations of the described and claimed aspects and embodiments. In the figures, each identical or nearly identical component that is illustrated in various figures is represented by a like numeral. For purposes of clarity, not every component may be labeled in every figure. In the figures:
FIG. 1 is an exemplary diagrammatic schematic of the inventive laser system for lithotripsy and the delivery system;
FIG. 2A illustrates a sequence of laser pulses with a constant peak power and uniform pulse period currently used in the laser lithotripsy of the known prior art;
FIG. 2B illustrates the pulse shape of a diode-pumped fiber or solid-state laser, known in the art;
FIG. 2C illustrates the pulse shape of a flash-lamp-pumped solid-state laser, known in the art;
FIG. 2D illustrates the pulse shape of a flash-lamp-pumped solid-state laser with spikes, known in the art;
FIG. 2E illustrates the pulse shape of a multi-head flash-lamp-pumped solid-state laser, known in the art;

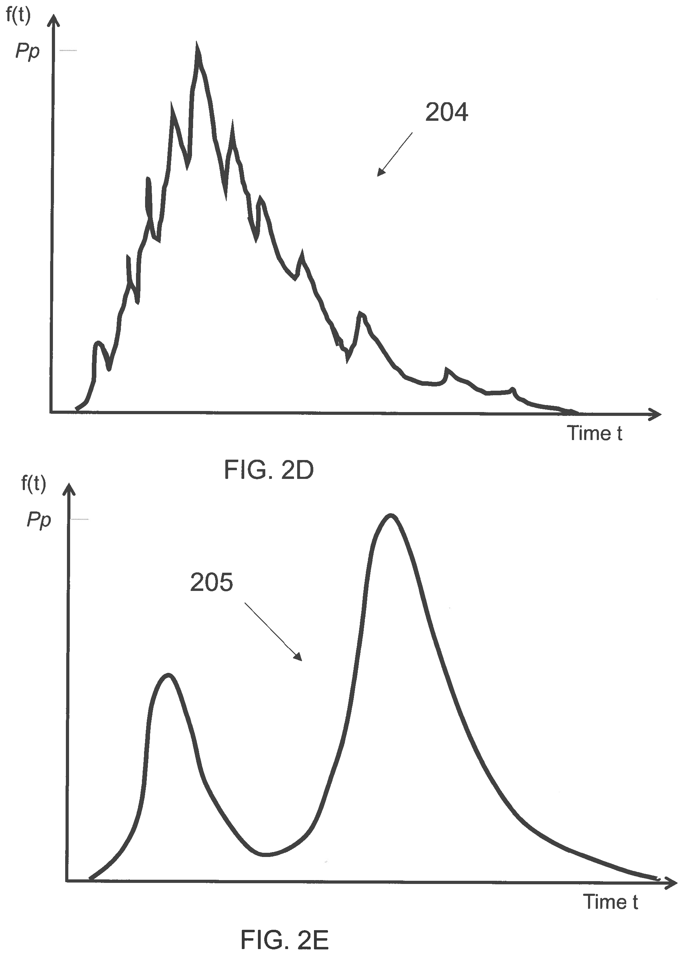

FIG. 2D illustrates the pulse shape of a flash-lamp-pumped solid-state laser with spikes, known in the art;

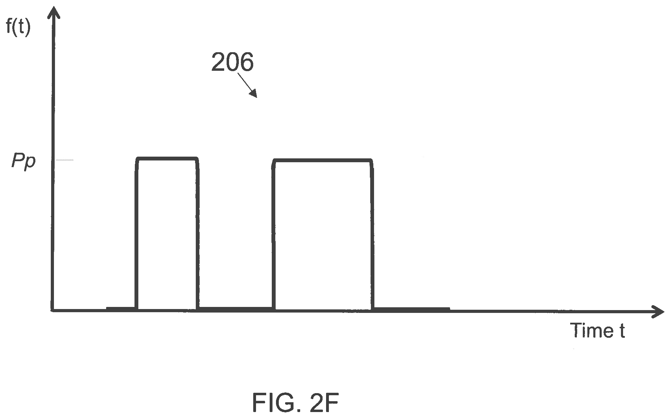

FIG. 2F illustrates the specially constructed pulse shape of a flash-lamp-pumped solid-state laser, designed to minimize energy losses in water between the distal end of the fiber and the target, known in the art;

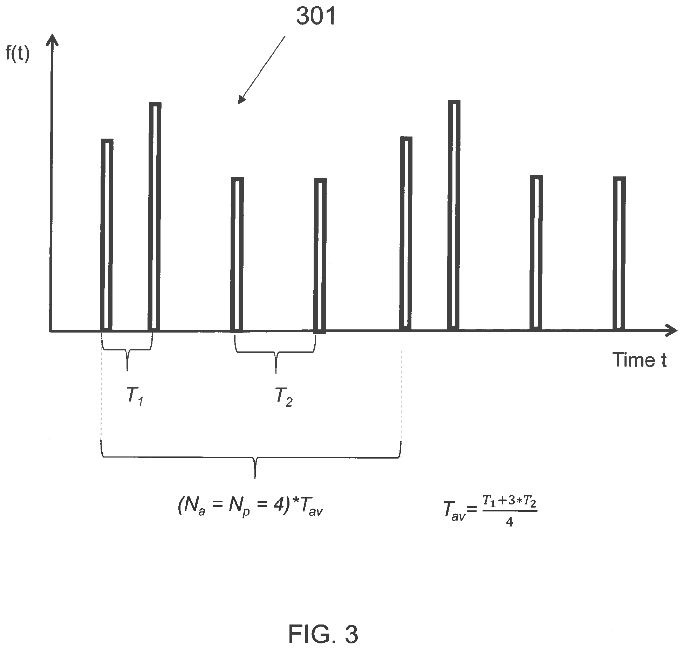

FIG. 3 illustrates an exemplary sequence of laser pulses emitted by the inventive laser system operating an amplitude-frequency modulated regime (AFMR) characterized by a periodically varied peak power Pp or energy E with a modulation period Na which is equal to a number of laser pulses. In the amplitude periodic pulse group; a periodically varied pulse frequency with a modulation period Np corresponding to a number of pulses in the frequency periodic pulse group;

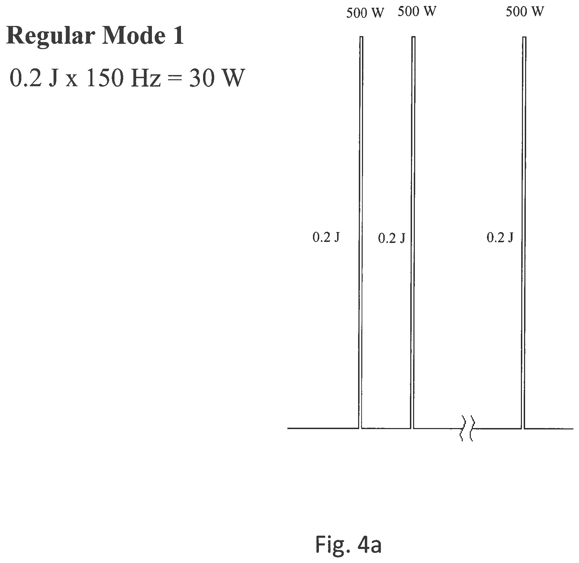

FIGs. 4A - 4B illustrate respective examples of laser pulse sequences known in the art and used in the dusting, fragmentation, and non-contact experiments as regular regimes;

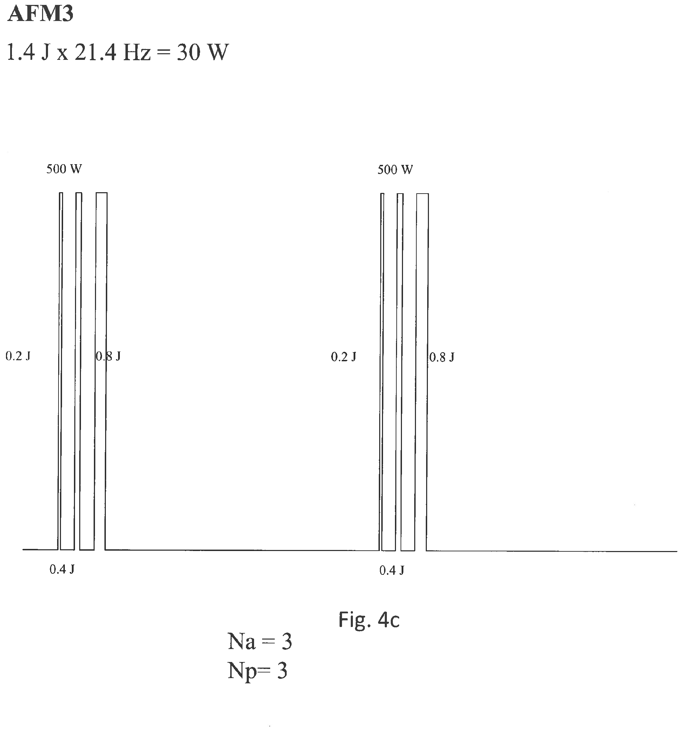

FIGs. 4C - 4E illustrate respective examples of laser pulse groups emitted by the inventive system operating in an amplitude-frequency modulation regime (AFMR) and used in dusting experiments;

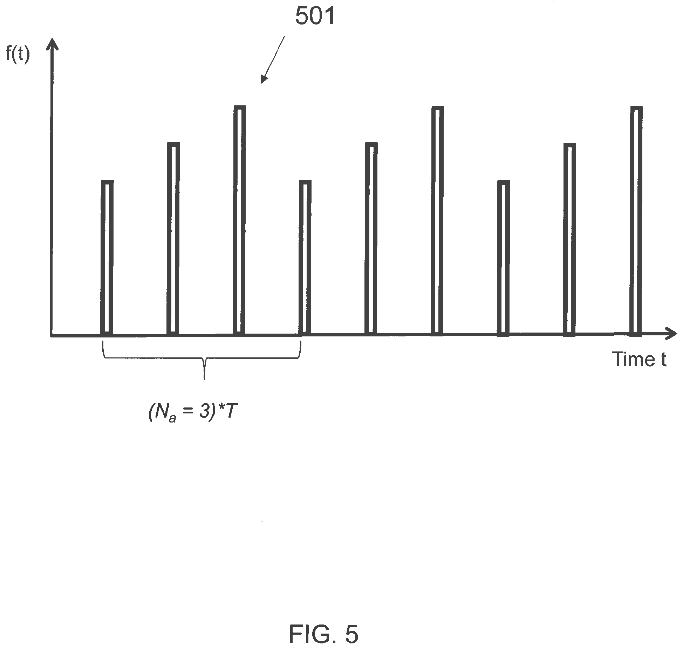

FIG. 5 is an example of an amplitude modulated regime (AMR);

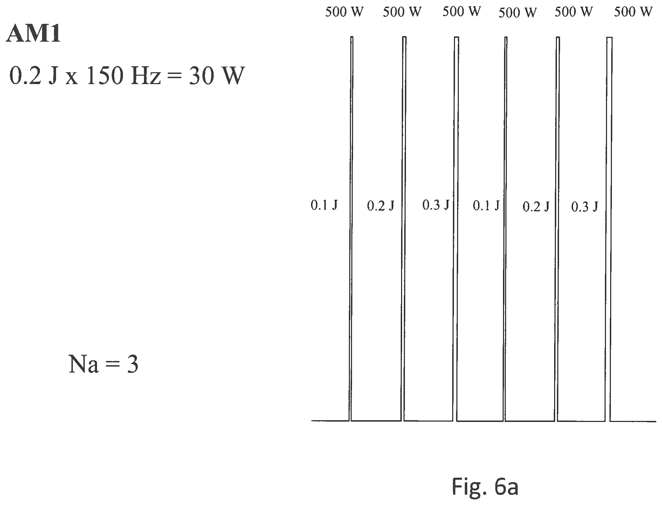

FIG. 6A - 6G are examples of amplitude periodic groups of laser pulses in the AMR characterized by different amplitude modulation period Na and used in contact-mode experiments;

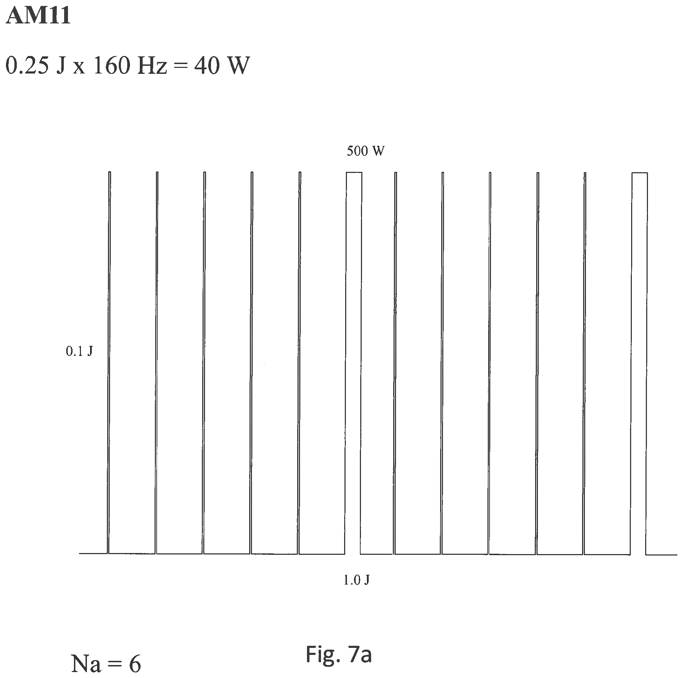

FIG. 7A - 7C illustrate further examples of amplitude periodic groups of laser pulses having respective modulation periods Na in accordance with the AMR and used in non-contact mode experiments;

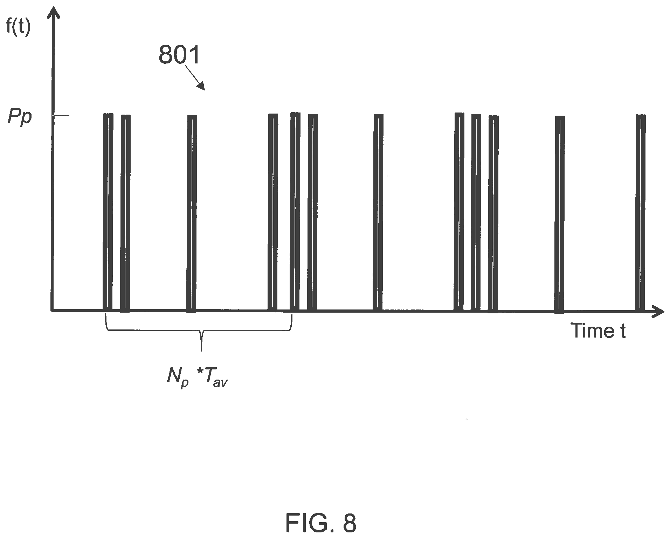

FIG. 8 is an example of a frequency modulated regime (FMR);

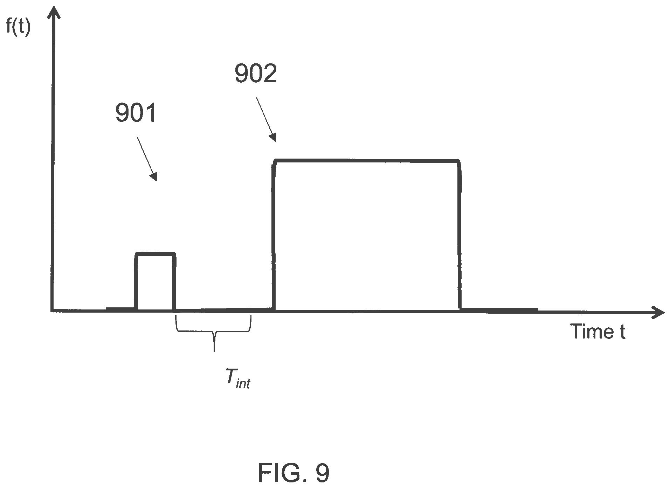

FIG. 9 is an example of a single laser pulse shaped with two temporally spaced sub-pulses which have different peak powers;

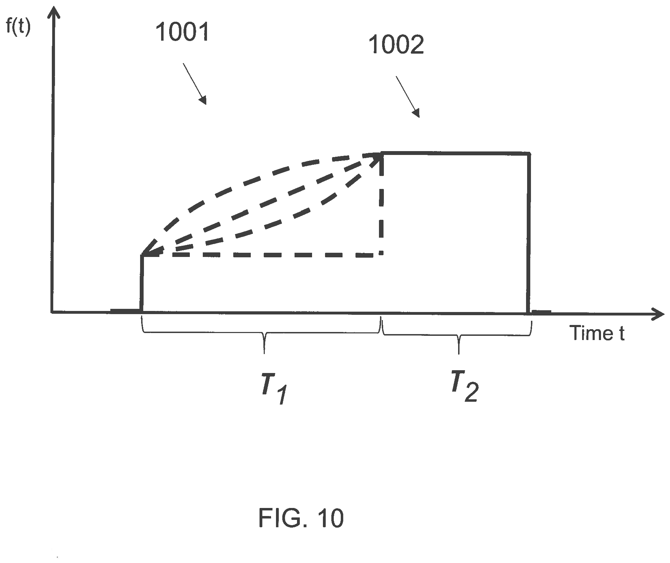

FIG. 10 is an example of a single laser pulse shaped with two adjacent sections; and

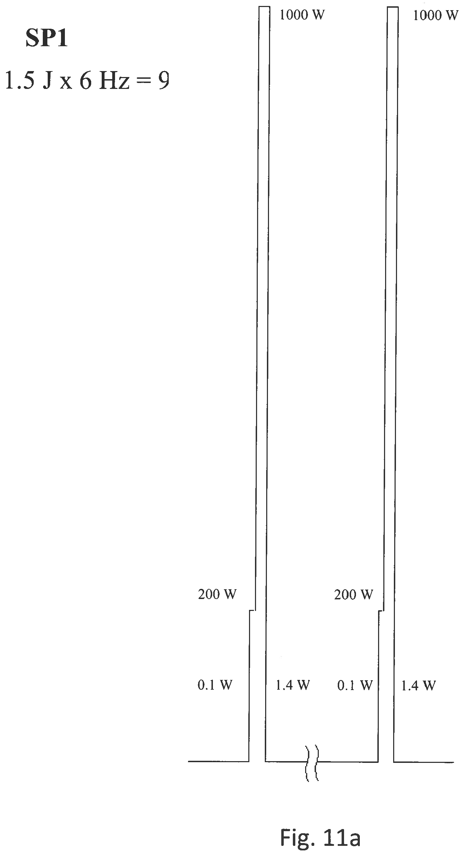

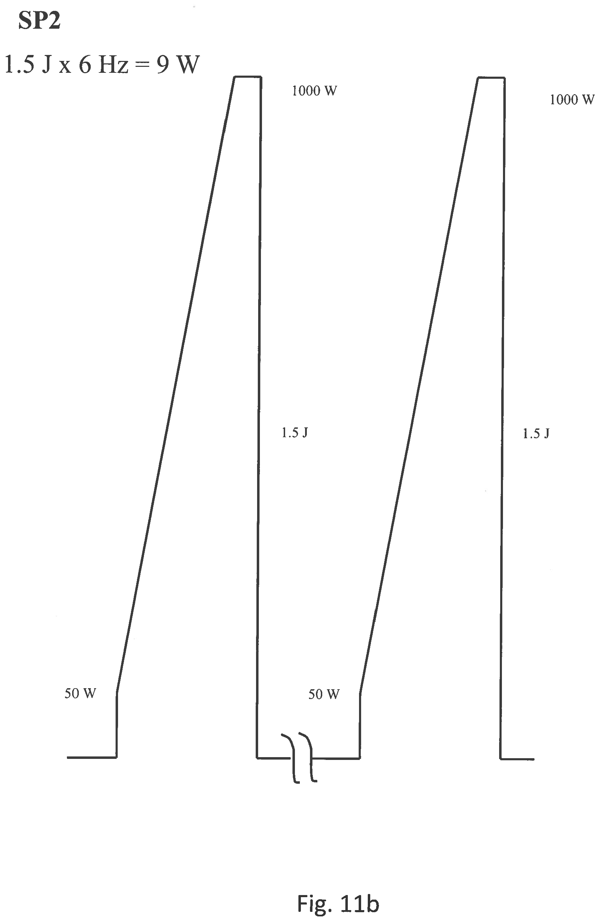

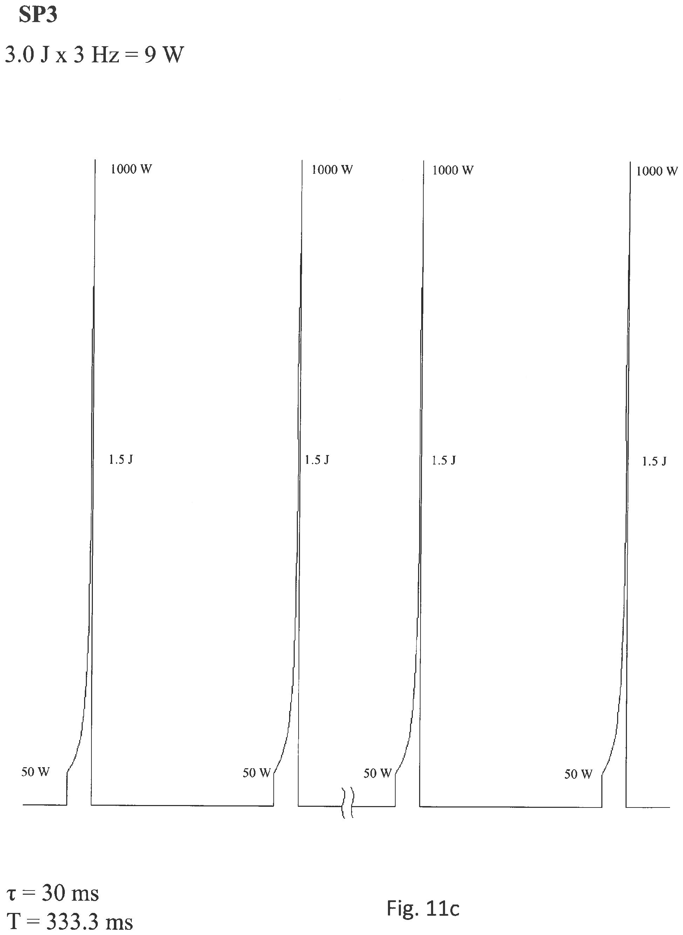

FIGs 11A, B and C are respective different laser pulse shapes used in fragmentation experiments.

### DETAILED DESCRIPTION

Reference will now be made in detail to embodiments of the invention. Wherever possible, same or similar reference numerals or letters are used in the drawings and the description to refer to the same or like parts or steps. The drawings are in simplified form and are not to precise scale. For purposes of convenience and clarity only, directional (up/down, etc.) or motional (forward/back, etc.) terms may be used with respect to the drawings. The term "couple" and similar terms do not necessarily denote direct and immediate connections, but also include connections through intermediate elements or devices.

The inventive concept of the present invention is based on providing a high ablation rate by increasing ablation efficiency while minimizing the retropulsion effect. The concept is realized by (a) modulation (periodic variation) of pulse energy E(n) or peak power Pp(n) or both, which is defined as AM, (b) modulation of pulse frequency (repetition rate) v(n) of a pulsing period T(n), which is defined as FM, (c) simultaneous amplitude and frequency modulation referred to as AFM, and (d) configuration of a specific pulse shape.

The first aspect of the invention - modulation of pulse peak power and/or energy - can be better understood in light of the following explanation. The present laser systems for treatment calulus in human or animal body temporal structure of laser power is a periodic pulse sequence (train) which can be described by the formula: $P \left(t\right) = Pp * f \left(t-T*n, n=0,1,2,…,\right.$ (1)
where P(t) is instantaneous laser power, Pp is peak power, f(t) is the individual pulse shape (profile), and T is the sequence period, which is inversely proportional to the pulse frequency: T=1/v. The energy per pulse is an integral of power: $E = INT \left(P\left(t\right), 0, T\right) = Pp * τ ,$ (2)
where τ is the effective pulse width.

The periodic pulse sequence can also be described by the formula: $P \left(t\right) = \left(E/τ\right) * f \left(t-T*n\right) ,$ (3)

The individual pulse shape (profile) f(t) is characterized by pulse width τ. For laser system for lithotripsy pulse width is much shorter than sequence period: τ <<T or τ <0.1T.

Conventional stone treatment techniques are based on regimes where Pp, T, v and E are set before treatment and kept constant during the treatment. Some laser systems can be configured with a dual pedal treatment parameter that is switchable between two sets of parameters with constant peak power, frequency and energy per pulse: Pp₁, T₁, v₁ ,E₁ and Pp₂, T₂, v₂ ,E₂.

### Amplitude Modulation (AM) in accordance with the invention:

In contrast to the known prior art, the amplitude modulation is defined here as a regime having a constant period T and frequency v and periodical variation of the peak power Pp: $P \left(t\right) = Pp \left(n\right) * f \left(t-T*n\right) , n = 0 , 1 , 2 , …$ (4)
or energy $P \left(t\right) = E \left(n\right) / τ \left(n\right) * f \left(t-T*n\right) ,$ (5)
where Pp(n)=Pp(n -Na) or E(n)=E(n-Na) or τ(n)=τ(n-Na) or its combinations, and Na is a positive integer number referred to as the period of the amplitude modulation. In the AM regime (AMR) of the inventive laser, all laser pulse sequences can be presented as a periodic sequence of a group of Na pulses with variable amplitude in the group (amplitude modulated periodic pulse group).

FIGS. 6A - 6G 2 and 7A - 7C illustrate the sequence of laser pulses with a different modulating period Na which are emitted from the inventive laser system operating in the AMR. These figured are discussed in detail below.

### Frequency Modulation(FM) in accordance with the invention

In contrast to the known prior art, frequency modulation as used in this application is defined as a regime having a constant peak power Pp or energy E and periodical variation of the frequency v or pulsing period T: $P \left(t\right) = Pp * f \left(t-T\left(n\right)*n\right) , n = 0 , 1 , 2 , …$ (6) or $P \left(t\right) = E / τ * f \left(t-T\left(n\right)*n\right) ,$ (7) where T(n)=T(n-Np) and Np is a positive integer number referred to as the period of frequency modulation. Frequency modulation implies frequency modulation because v(n)=1/T(n)=v(n-Np). In the FM regime (FMR), all laser pulse sequences can be presented as a periodic sequence of a group of Np pulses with a variable period between the pulses in the group (frequency modulated periodic pulse group).

FIG. 8 illustrates the inventive laser system operating in the FMR.

### Amplitude Frequency Modulation (AFM) in accordance with the invention:

Based on the AM and FM defined in accordance with the invention, an amplitude-frequency modulation is defined as a regime having simultaneous periodic variation of peak power Pp (or energy E) and frequency v (and period T): $P \left(t\right) = Pp \left(n\right) * f \left(t-T\left(n\right)*n\right) , n = 0 , 1 , 2 , …$ (80
or energy $P \left(t\right) = E \left(n\right) / τ \left(n\right) * f \left(t-T\left(n\right)*n\right) ,$ (9)
where Pp(n)=Pp(n -Na) or E(n)=E(n-Na) or τ(n)=τ(n-Na) and T(n)=T(n-Np).

FIGs. 3 and 4A - 4C illustrate the inventive system operating in the AFMR and are discussed in detail below.

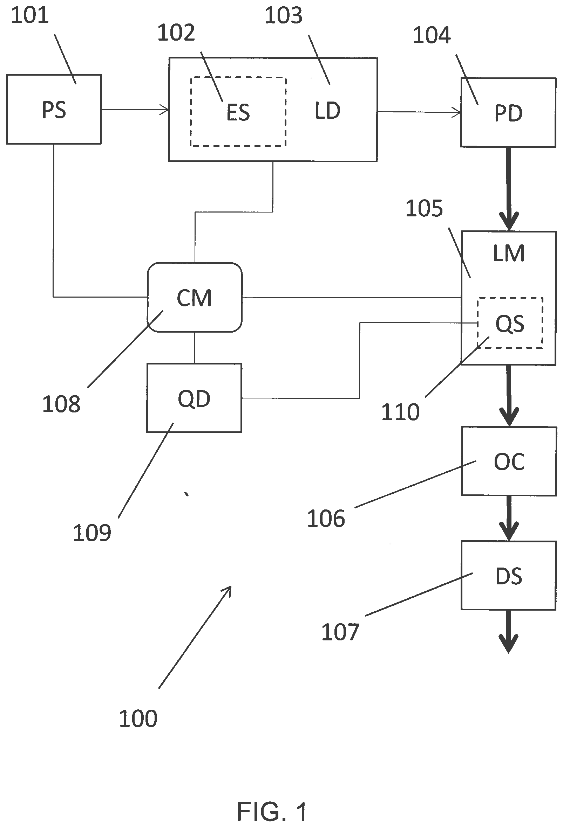

Turning to FIG. 1 illustrating a diagrammatically shown exemplary inventive laser system 100 that those skilled in the art immediately realize that structurally this system is relevant to the discussion of both inventive aspects and can be embodied using various types of laser technology. However, a preferred embodiment is based on a particular class of laser technology, specifically, a pulsed laser technology including a pulsed laser 104 operating either in a free running mode or Q-switch mode. In either mode of the laser operation and regardless of the laser type, inventive laser system 100 is configured with a pump 103 energizing laser 104.

Typically, pump 104 is configured with one or more diode lasers. In optimum embodiment pulsed laser technology implies the use of an energy-storing device (e.g., electrical capacitors, inductors, or combinations of these).

The power supply 101 supplies power to the system, optionally energy-storing device 102 stores a sufficient amount of energy necessary to form a laser pulse. The laser driver 103 of pump 104 forms an electrical pulse of specified characteristics in response to a control signal from control module 108. The electrical pulses are received by one or more diodes of pump 104 which form an optical pulse necessary to pump the laser medium in a laser cavity 105. The Output of the laser medium is coupled to the delivery system 107 which is considering as external to laser system through the optical coupler 106.

The whole system is controlled by control module CM (108), providing calibration curves or tables (defining characteristics of the electrical pulse necessary to achieve desired optical output) which are contained the control signal, timing and safety features. Instead of pumping diodes, other devices, such as e.g. flash lamps, can be used as pumping sources.

Alternatively, the laser medium itself can be used as an energy-storing device. In this configuration the pulse formation is achieved through application of an internal optical modulator of cavity losses as Q-modulation devices such as acoustic-optical, electro-optical or passive modulators.

For the purposes of the present invention, the following terms are defined as:
the laser pulse is an output of inventive laser system 100 generated by direct modulation of the diode current by laser driver 103 or a single charge-discharge cycle of the energy-storing device 102,
the sequence of laser pulses is an output of the laser system generated by multiple direct single modulation or single charge-discharge cycles of the energy-storing device 102.

Thus the modulation of the pulse sequence in accordance with the one aspect of the invention is achieved through setting the desired peak power Pp or the pulse energy E for the AM or the interval T between two consecutive pulses in the FM regime by control module 108. The pulse shaping in accordance with the other inventive aspect is achieved through forming the desired temporal structure of a pulse in laser driver 103.

Based on the above-discussed equations 4 - 9, which mathematically describe the inventive AM, and combined AM and FM, laser system 100 operate in accordance with first aspect by controlling the temporal structure of the laser emission is controlled through*:*
a) Modulating the peak power Pp or pulse energy E for a number of pulses in a sequence of pulses Pp(n)=Pp(n-Na) where Na is the period of the amplitude modulation;
b) Modulating the period T (and, respectively, the frequency v) of the pulse sequence for a number of pulses T(n)=T(n-Np), where Np is the period of the frequency modulation;
c) Combining the modulation modalities described in a), b) and c) above; for example, simultaneous modulation of the amplitude and the frequency (amplitude-frequency modulation).

All three types of modulation (amplitude, frequency, and amplitude-frequency) as well as adjusting the individual pulse shape are used in various aspects of the present invention. Diode-pumped fiber (preferable) and solid state lasers can be modulated by controlling the current of the pumping diodes and allow to modify laser parameters by varying the current on pumping diodes. Preferably, the diode current should be varied in the range between threshold current Iₜₕ of the diode pumped laser generation and some maximal current, which is below the level of saturation of laser power as function of diode current Ist. In this range the laser power is almost linearly dependent on the diode current and different laser temporal structures can be produced by programming the pumping current of laser driver.

Main propose of the invention is to increase speed of stone dusting or fragmentation without compromising or (preferably) increasing safety profile. This can be achieved by proper pulse shaping or by modulating sequence of pulses (with one or more of modulation modalities described above). Such modulation can be optimal for each mode of treatment such as contact dusting or fragmentation and for non-contact dusting.

In the case of contact dusting, the desired end result is fragmentation of stones into small particles smaller than 1 mm, preferably smaller than 0.5 mm, and most preferably smaller than 0.25 mm. In current laser systems, this regime requires continuous movement of the fiber across the stone surface using a relatively low energy per pulse (0.025 - 0.3 J). To compensate for the low ablation volume per pulse due to the low energy per pulse, the repetition rate should be as high as possible, while keeping the average power Pa=E*v within the safe limits to avoid thermal damage of soft tissues due to water heating in the urinary tract. This safety limit of Pa max depends on the water irrigation rate and the total treatment time. The temporal structure of the laser output can be optimized to achieve higher ablation speed with equal average power through laser pulse shaping and/or modulating the sequence of laser pulses, and at the same time decreasing or at least keeping the same level or retropulsion. Ablation efficiency and the retropulsion effect are the results of the combination of many factors which include but are not limited to:
1. Pulse energy, pulse peak power or pulsewidth, rep rate
2. Beam diameter, which depends on the fiber core diameter
3. The distance between the fiber end and the stone
4. The speed of fiber movement. This factor is related to the number of effective pulses applied to one spot. This number can be estimated by formula K=(d/2v)*v, where d is the beam diameter on the stone surface and v is the speed of movement of the fiber. The efficiency of ablation decreases with the increase of K because of the increasing distance between the fiber end and the bottom of the laser crater, the shading effect from the products of ablation, the loss of water at the bottom of laser crater, and other factors
5. Oscillation of the fiber induced by laser pulsing. The fiber can oscillate in scope with a certain amplitude. These oscillations are induced by force from the laser induced bubble formation in water, the electrostriction effect, and other mechanisms. Fiber oscillation effectively decreases the number of pulses applied to one spot K, which can increase the ablation efficiency.
6. Size and shape of a stone

Pulse modulation works in combination with all the listed factors but can lead to different effects. For example, a periodical increase of laser energy from minimum to maximum can compensate for the decrease of ablation efficiency while lasing with constant energy in one spot with the same frequency and average power and fiber movement speed. Provided below is an experimental comparison of stone ablation and retropulsion speed using constant pulse energy and peak power and frequency with different regimes of amplitude (regular regimes, which represent current regime of treatment stone), amplitude and amplitude-frequency modulation prove advantages of the proposed regimes for contact and non-contact modes.

### Characterization of Temporal Regimes of Laser Lithotripsy

The overarching goal of optimizing laser lithotripsy is to accelerate the procedure, to ensure breakage of stones into fragments of desired size, and to minimize the incidence of side effects. Among the parameters relevant to this goal, the two most important are the efficiency of stone ablation and the magnitude of the retropulsion effect. For the purpose of comparing various temporal regimes of laser emission, we use the following metrics: 1) Efficiency of stone ablation *Ka*, defined as *Va*/*Et*, where *Va* is the total ablated volume, Et is the total laser energy [mm³/J]; 2) Critical retropulsion velocity *Vr*, defined as the velocity of retropulsion in the first instance of laser treatment [mm/s]; 3) Absolute quality of the temporal regime *Qa*, defined as *Ka*/*Vr* [mm²/W] which increases with ablation efficiency and decreases with retropulsion; 4) Relative quality of the temporal regime *Qr*, defined as *(Qa)*/*(Qa)ref*, where index *ref* refers to a reference regular regim [dimensionless]; 5) Time of stone cracking which defines as time to crack stone in fragmentation mode

The following experimental techniques were used to characterize and compare various temporal regimes:

### Scanning Experiments

### Equipment:

1) Tm-fiber laser with wavelength 1.94 µm, peak power up to 1000 W
2) Delivery fiber with a core diameter of 200 µm
3) 2D-motorized stage
4) High speed camera (Phantom Miro M310 by Phantom Vision Research)
5) Fiber holder
6) Mechanical profilometer (Contracer^{®} by Mitutoyo, Kawasaki, Japan)

### Materials and methods:

All experiments were performed with artificial stone phantoms. Stones were produced using BegoStone powder (Bego GmbH, Bremen, Germany) with a powder to water ratio of 5:1. Samples were cut into slabs having dimensions of 60x40x8 mm. Stones were soaked in water for 24 hours prior to laser exposure.

Ablation efficiency and the retropulsion effect were measured on two different setups.

First, the stones were placed in a container with water (for adequate positioning of the setup, two bubble levels were used.). The fiber holder was mounted on a 2-D-motorized stage. Linear craters of 30-mm-length were created using 1-D horizontal fiber movement with a speed of 6 mm/s, which represents a typical clinical speed of scanning. Laser parameters were varied according to the tables below. Measurements of the cross-sectional area, depth and width of the crater were performed using the mechanical profilometer. The ablation rate and efficiency were computed using cross-sections of profiles multiplied by the speed of scanning and divided by the average laser power. During scanning, the distance between the fiber end and flat stone surface was kept at about 0.2 +/- 0.1 mm.

Second, the retropulsion effect was measured for the same laser parameters. To measure the magnitude of stone displacement, two linear rulers were attached along the long sides, forming a 90⁰ groove. The groove was submerged in a water bath. For adequate positioning of the setup, two bubble levels were used. Stone samples (5x5x5 mm cubes) were placed into the groove. The fiber was introduced through a holder located at a hole in the sidewall of the setup. The fiber tip was brought into contact with the stone center. For capturing the stone movement, a high-speed (1000 frames per second) camera (Phantom MIRO M310, Phantom Vision Research, USA) was used. The stone's movement was analyzed during the first 0.5 second of the exposure. The movement of the stone as a function of time was quantified using Image software and the speed of stone movement in the initial moments of lasing was computed as the slope of such function at the start of lasing.

### Bubble-characterization experiments

### Equipment:

1) Tm-fiber laser with wavelength 1.94 µm, peak power up to 1000 W
2) Fiber with a core diameter of 200 µm
3) Quartz cuvette
4) Laboratory stand
5) Halogen lighting system
6) High speed camera Phantom Miro M310

### Materials and methods:

The fiber holder was attached to a laboratory stand. The fiber was placed in a quartz cuvette filled with water. Using a high-speed camera, a video bubble formation by a single pulse was recorded for various laser parameters (energy and peak power laser in the range from 0.025 mJ to 0.4 J and from 100 to 500 W, respectively). The frame rate of the camera was 120,000 frames per second, the exposure time was 7 µs. Halogen lighting system was used to illuminate the scene. The recorded video was used to evaluate the growth time of a bubble up to 1 mm, up to 2.5 mm, and up to the maximum dimensions of the bubble. The bubble length was quantified using ImageJ software.

### Non-contact mode

### Equipment:

1) Tm-fiber laser with wavelength 1.94 µm, peak power up to 1000 W
2) Fiber with a core diameter of 200 µm
3) Flexible endoscope
4) Two glass cuvettes

### Materials and methods:

The experimental setup included a specially constructed inner cuvette 13 mm in diameter, with 0.25 mm holes drilled in the walls at a height of 40 mm. The laser treatment was conducted through a flexible endoscope. The flow of water through the flexible endoscope was 10 ml/min. The inner cuvette was placed into an outer cuvette, which collected the outgoing water with suspended dust particles smaller than 0.25 mm evacuated by the water flow through the side holes during the lithotripsy. BegaStone balls with a radius of 2 mm were used in this study as stone phantoms. 5 balls were used for each laser parameter. Lithotripsy was performed for a duration of 2 min and 40 seconds. After lithotripsy, the fragments remaining in the inner cuvette were weighed. The mass of dust was determined as the difference between the initial mass of the balls and that of the remaining fragments. The ablation rate was defined as the ratio between the mass of dust and duration of treatment.

### Drilling and Cracking

### Equipment:

1) Tm-fiber laser with wavelength 1.94 µm, peak power up to 1000 W
2) Fiber with a core diameter of 200 µm
3) Fiber holder
4) Glass cuvette
5) Stopwatch

### Materials and methods:

The experiment was performed with artificial stone phantoms. Stones were produced using BegoStone powder (Bego GmbH, Bremen, Germany) with a powder to water ratio of 5:1. The stones were sized at 5x2.5x2.5 mm. The stones were soaked in water for 24 hours prior to laser exposure. The stone samples were placed in a glass cuvette filled with water. The stones were drilled through with various parameters of laser radiation in such a way that the fiber was always in contact with the stone in the center of 5 x2.5 mm side to imitate a fragmentation regime of treatment. During the experiment, the cracking time stone samples on two fragments was recorded using a stopwatch. After that, the retropulsion was evaluated with the same laser parameters using the set-up described in the Scanning experimental section above.

All measuring where repeated 3 time for every setting point, mean value and standard deviation were calculated.

### Amplitude-Frequency modulation(AFM)

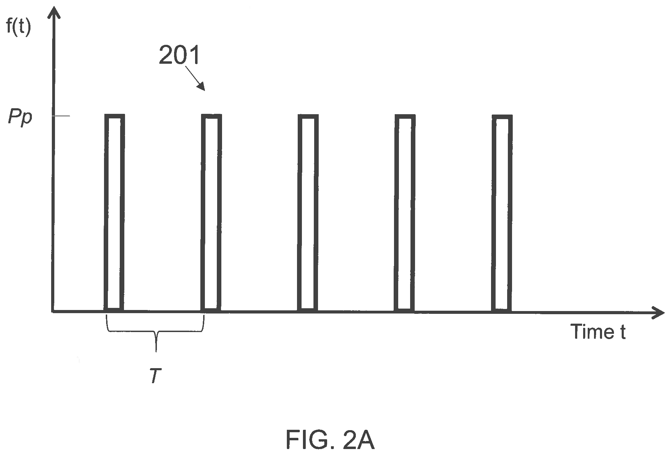

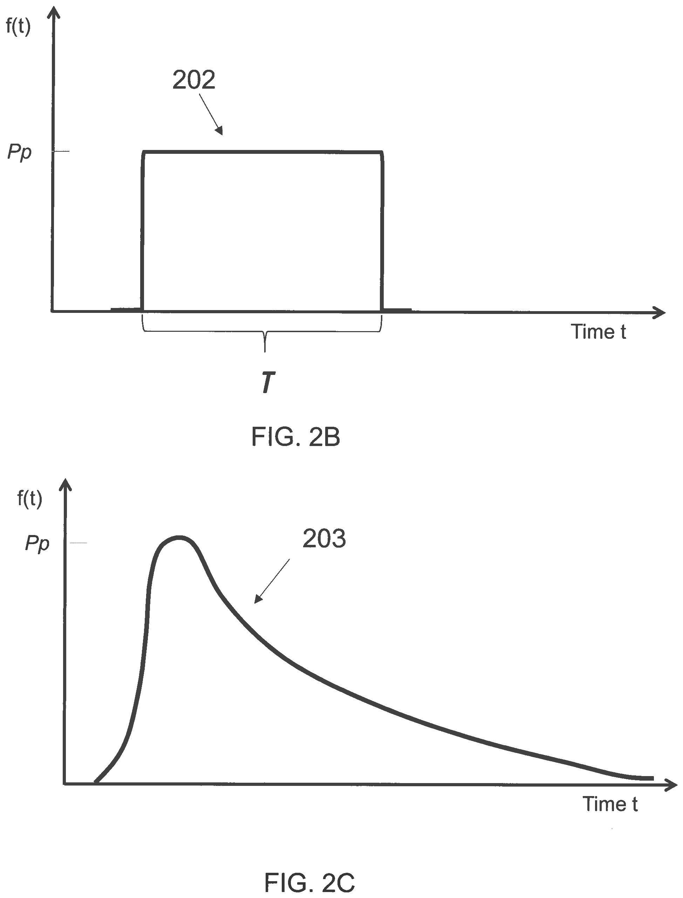

The general case of the laser pulse sequence currently used in current laser lithotripsy is illustrated by FIG. 2. Here, the sequence is characterized by the constant magnitude (peak power) *Pp,* the constant pulse energy *E*, and the constant period *T* of each single pulse (FIG. 2A). The single pulse (201) shape, on the other hand, can vary between a simple quasi-rectangular pulse 202 (FIG. 2B), which is typical for diode pumped lasers; pulse shape 203 (FIG. 2C), characteristic for the flash lamp pumped lasers, with steep rising edge and long tail; pulse shape 204 (FIG 2D), which is also common for flash lamp pumped solid state lasers, where smooth general shape is modulated with irregular micro-pulses or spikes due to relaxation oscillation of the laser; or compound pulse shape 205 (FIG. 2D), appearing in multi head flash lamp solid state laser systems. Other pulse shapes are known in the art, such as, for example, pocket of regular micro-pulses [Blackmon RL, Fried NM, Irby PB. Enhanced thulium fiber laser lithotripsy using micro-pulse train modulation. Journal of biomedical optics. 2012 Feb;17(2):028002] or a pulse consisting of two sub-pulses 206 (FIG. 2E), with has leading lower energy sub-pulse followed by a higher energy trailing sub-pulse 206 after an interval of 100 to 200 µs [US Patent 5 321 715].

In contrast, the present invention emphasizes various advantages and benefits brought about by varying one or more of the quantities *Pp, E,* and *T* according to Eqs (4-9). Amplitude-frequency modulations the most general type of modulation covered by the present invention. An example of AFM is shown in Fig. 3. Here, the amplitude modulation period Na equals the frequency modulation Np and equals 6. The AFM may be characterized by an average group period Tav, defined as: $Tav = \frac{1}{{N}_{g}} {\sum }_{i = 1}^{{N}_{g}} {T}_{i} ,$ (10) where Ng is the number of pulses in the periodic group, Ti is the period of the i-th pulse. AFM is beneficial for both contact and non-contact modes of treatment. The preferred AFM parameters are as follows:
1. Scanning mode
   Preferable parameters:
   Wavelength 1.81-2.2 µm, more preferable 1.908-1.98 µm
   Peak power Pa= 250- 5000 W, more preferable 400 - 1000 W
   Energy per pulse 0.01 - 2 J, more preferable 0.05 - 0.5 J
   Pulse rep rate v=5- 3000Hz/Period T=0.00033-0.2s, more preferable 50 - 1000 Hz, 0.001-0.02s
   Na=2-10
   Np=1-100

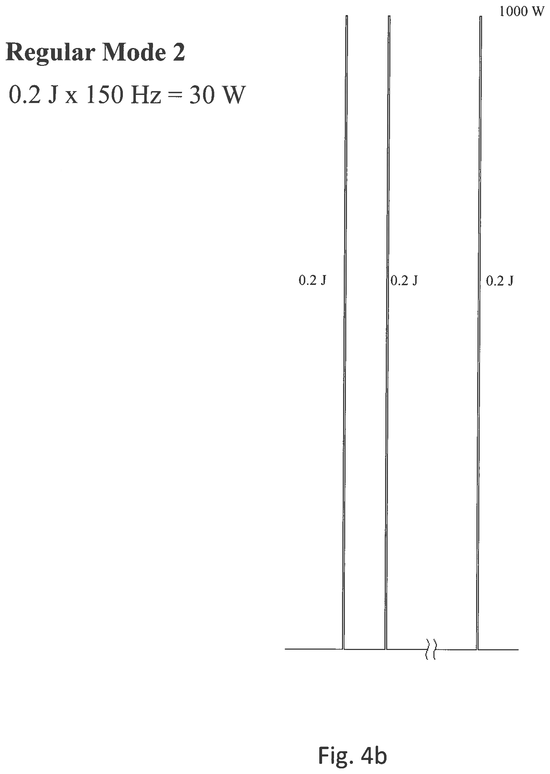

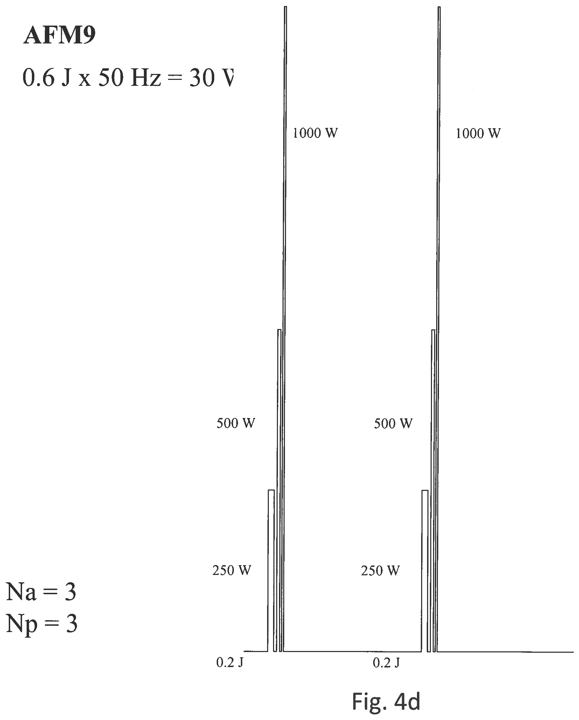

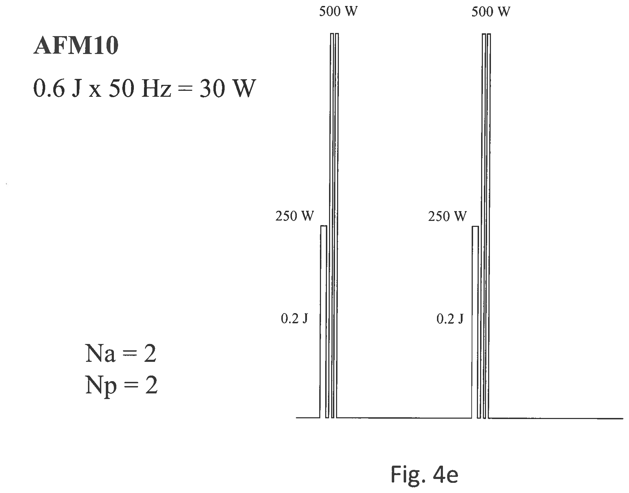

These settings are exemplified by Fig.4 and Table 1. All experiments were performed with identical average power 30 W to provide the same soft tissue safety profile.

**Table 1. Examples of AFM sequences for contact/scanning mode of lithotripsy.**

| ***Sequence name*** | ***Figure of temporal structure*** | ***Modulated amplitude and frequency*** | ***Rep rate, Hz*** | ***Amplitude period, Na*** | ***Ablation efficiency, mm3*/*J*** | ***Ablation depth, mm*** | ***Retropulsion speed, mm*/ *s*** | ***Relative sequence quality Qr (WRT Reference)*** |
|---|---|---|---|---|---|---|---|---|
| | | | | | | | | |
| Regular1 peak power 500 W (Reference) | 4a | None | 150 | | 0.03 ± 0.01 | 0.33 ± 0.02 | 20.8 ± 0.65 | 1 ± 0.2 |
| Regular2 peak power 1000 W | 4b | None | 150 | | 0.06 ± 0.02 | 0.55 ± 0.07 | 31.1 ± 0.9 | 1.4 ± 0.3 |
| | | | | | | | | |
| AFM3 | 4c | Period and Energy | 21.4 | 4 | 0.11 ± 0.05 | 0.76 ± 0.18 | 23.9 ± 0.9 | 3.1 ± 0.4 |
| AFM9 | 4d | Period and Peak Power | 50 | 4 | 0.10 ± 0.02 | 0.79 ± 0.04 | 29 ± 2.16 | 2.3 ± 0.3 |
| AFM10 | 4e | Period and Peak Power | 50 | 4 | 0.10 ± 0.03 | 0.72 ± 0.05 | 26 ± 0.4 | 2.3 ± 0.2 |

The table shows that AFMM regimes significantly increase ablation efficiency (up to 3.1 times) and depth of ablation without increasing retropulsion compared with the regular (unmodulated) regimes with 500 W and 1000 W of peak power.

### Contact/Fragmentation mode

### Preferable parameters:

Wavelength 1.81-2.2 µm, more preferable 1.908-1.98 µm
Peak power Pa= 100 - 2000 W, more preferable 250 - 1000 W
Energy per pulse 0.2 - 10 J, more preferable 0.5 - 5 J
Pulse rep rate *v*=1- 300Hz/Period T=0.0033-1s
Na=2-10
Np=1-100

### 2. Non-contact (popcorning) mode

### Preferable parameters:

Wavelength 1.81-2.2 µm, more preferable 1.908-1.98 µm
Peak power Pa= 500- 3000 W, more preferable 500 - 2000 W
Energy per pulse 0.05 - 1 J, more preferable 0.05 - 0.5 J
Pulse rep rate *v*=10- 1000Hz/Period T=0.001-0.1s
Na=2-100
Np=1-100

### Amplitude Modulation

Amplitude modulation is a particular case of AFM, when the pulse period remains constant. A typical case of amplitude modulation (AM) is shown in Fig. 5. Here, the magnitude (*Pp* or *E*) varies with the period Na=3, whereas the period T between the individual pulses stays constant. The group of pulses 501 in the time interval *T*Na* is referred to as the amplitude modulated periodic pulse group.

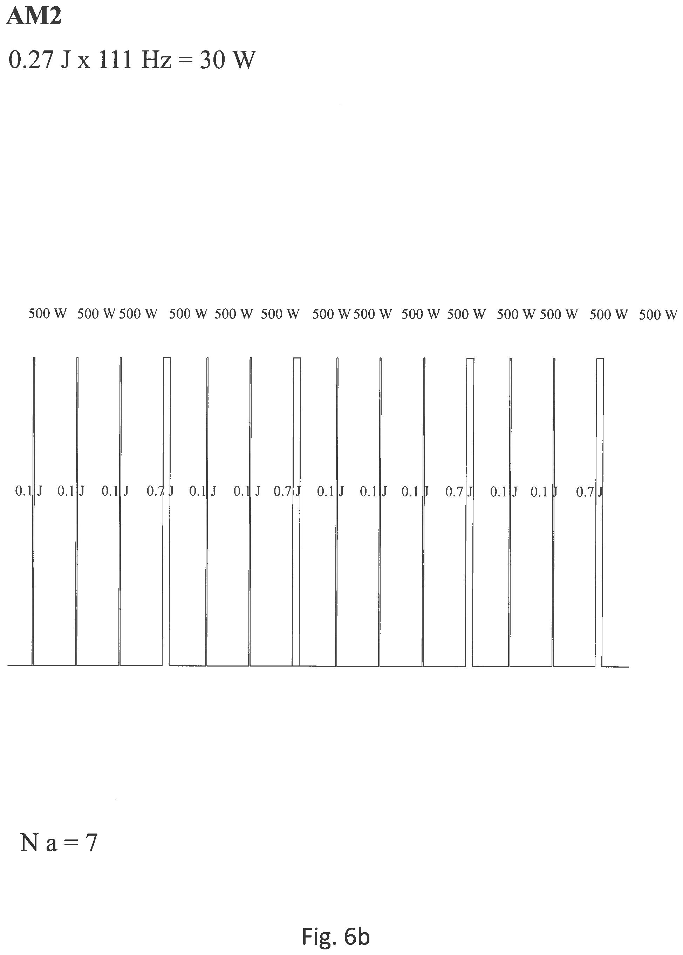

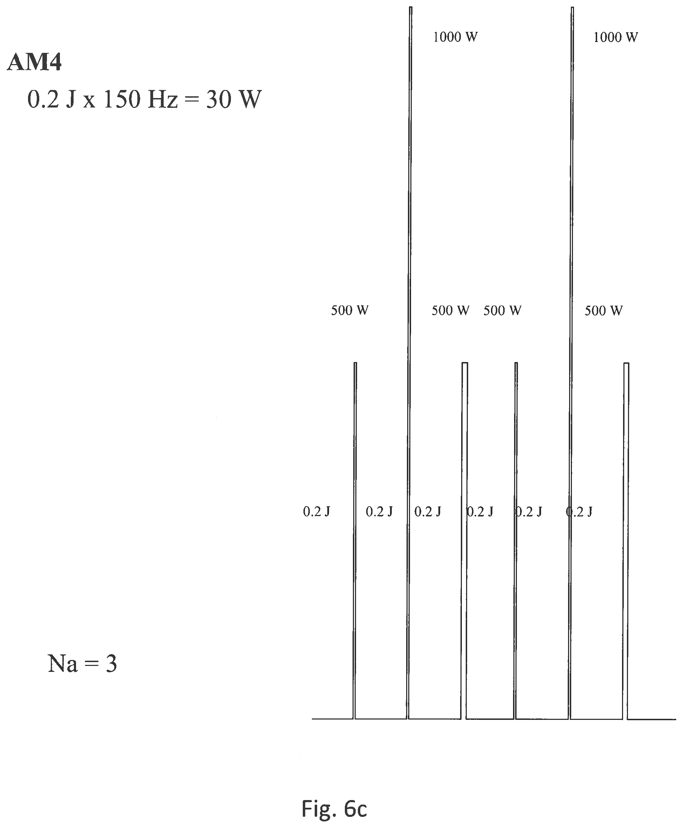

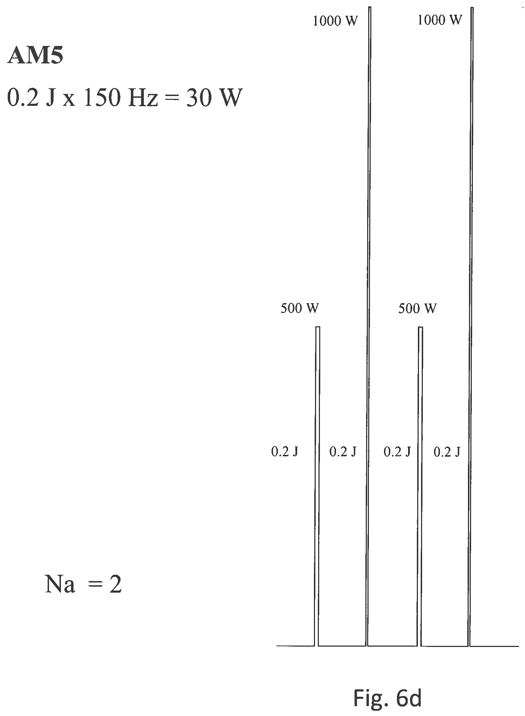

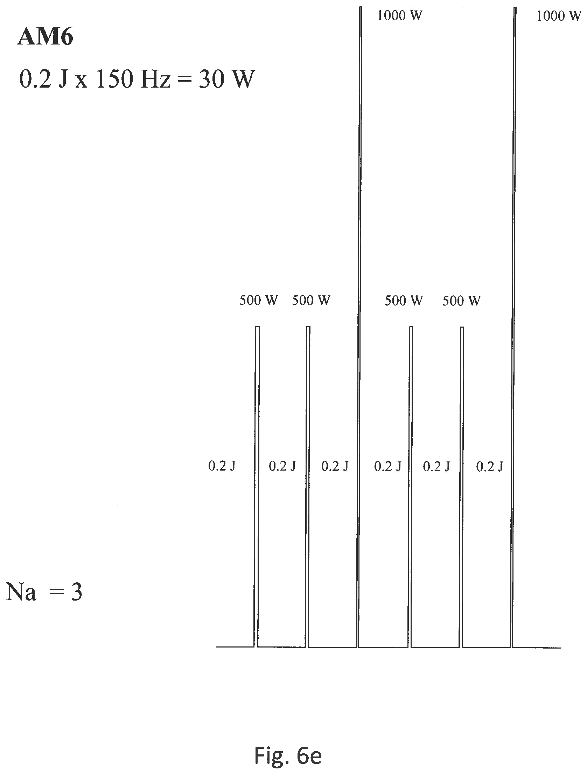

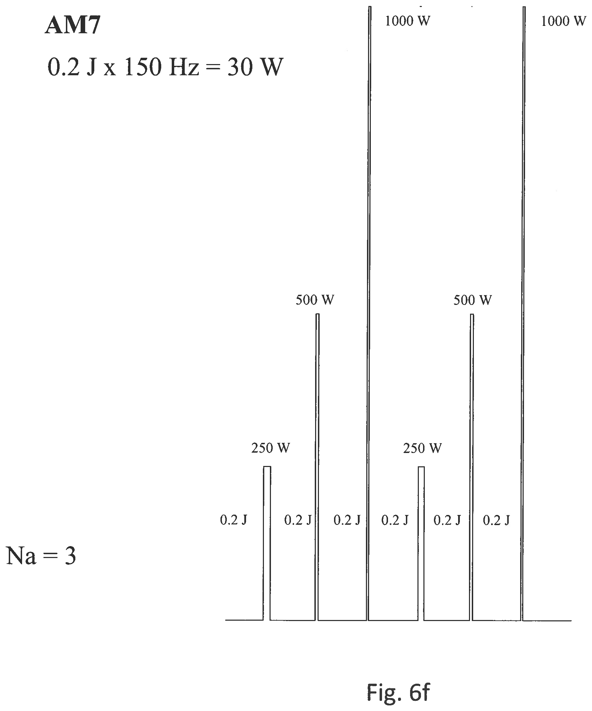

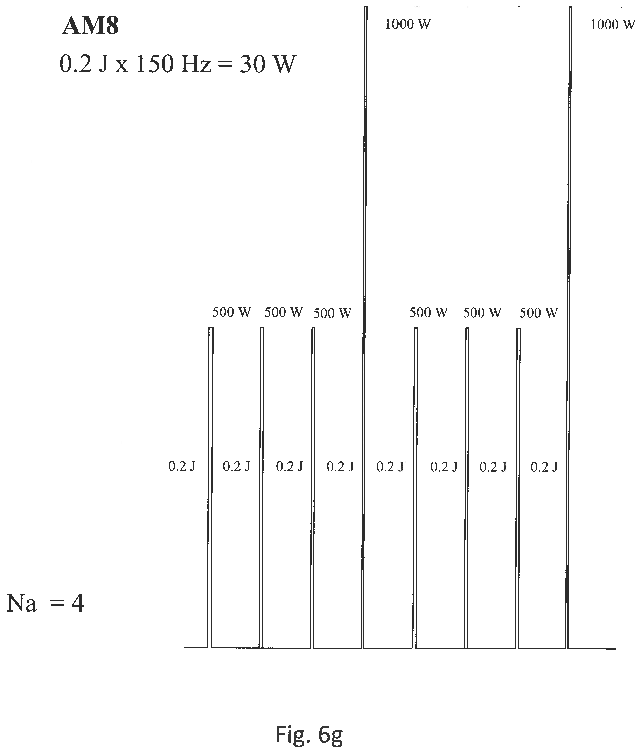

Many varieties of the pulse groups are possible. Some are illustrated by Fig. 6(a-g). As can be seen from the Fig. 6, both *Pp* and *E* can be varied within the framework of AM.

AM can be beneficial for both principal modes of the laser lithotripsy (i.e., contact and non-contact). Summarized below are preferable regimes and illustrative examples:

### 1. Contact/Scanning mode

Preferable parameters:
Wavelength 1.81-2.2 µm, more preferable 1.908-1.98 µm
Peak power Pa= 250- 3000 W, more preferable 400 - 1000 W
Energy per pulse 0.02 - 2 J, more preferable 0.05 - 0.5 J
Pulse rep rate *v*=5- 3000Hz/Period T=0.00033-0.2s, more preferable 50 - 1000 Hz, 0.001-0.02s
Na=2-10

These settings are exemplified by Fig.6 and Table 2.

**Table 2. Examples of AM sequences for contact/scanning mode of lithotripsy.**

| ***Sequence nam e*** | ***Temporal structure, Figure*** | ***Modulated amplitude*** | ***Rep rate, Hz*** | ***Amplitude period, Na*** | ***Ablation efficiency, mm3*/*J*** | ***Ablation depth, mm*** | ***Retropulsion speed, mm*/ *s*** | ***Relative sequence quality Qr (WRT Reference)*** |
|---|---|---|---|---|---|---|---|---|
| | | | | | | | | |
| Regular1 with peak power 500 W (Reference) | 4a | None | 150 | | 0.03 ± 0.01 | 0.33 ± 0.02 | 20.8 ± 0.65 | 1 ± 0.2 |
| Regular2 Regular1 with peak power 1000 W | 4b | None | 150 | | 0.06 ± 0.02 | 0.55 ± 0.07 | 31.1 ± 0.9 | 1.4 ± 0.3 |
| | | | | | | | | |
| AM1 | 6a | Energy | 150 | 3 | 0.07 ± 0.01 | 0.61 ± 0.16 | 23.7 ± 0.8 | 2.1 ± 0.3 |
| AM2 | 6b | Energy | 150 | 4 | 0.1 ± 0.02 | 0.77 ± 0.08 | 22.1 ± 1.0 | 3.1 ± 0.4 |
| AM4 | 6c | Energy and Peak Power | 150 | 3 | 0.08 ± 0.01 | 0.54 ± 0.1 | 30.2 ± 0.3 | 1.8 ± 0.2 |
| AM5 | 6d | Peak Power | 150 | 2 | 0.07 ± 0.01 | 0.71 ± 0.06 | 21.8 ± 0.46 | 2.1 ± 0.3 |
| AM6 | 6e | Peak Power | 150 | 3 | 0.06 ± 0.01 | 0.66 ± 0.1 | 21.2 ± 0.47 | 2.1 ± 0.3 |
| AM7 | 6f | Peak Power | 150 | 3 | 0.07 ± 0.01 | 0.71 ± 0.04 | 18 ± 0.15 | 2.6 ± 0.4 |
| AM8 | 6g | Peak Power | 150 | 4 | 0.06 ± 0.01 | 0.63 ± 0.07 | 20.9 ± 0.91 | 2.1 ±0.3 |

These data suggest that the amplitude modulation may increase the regimen quality more than 3-fold.

### 2. Contact/Fragmentation mode

Preferable parameters:
Wavelength 1.81-2.2 µm, more preferable 1.908-1.98 µm
Peak power Pa= 100 - 20000 W, more preferable 250 - 3000 W
Energy per pulse 0.2 - 20 J, more preferable 0.5 - 10 J
Pulse rep rate *v*=1- 500Hz/Period T=0.0002-1s
Na=2-10

### 3. Non-contact (popcorning) mode

Preferable parameters:
Wavelength 1.81-2.2 µm, more preferable 1.908-1.98 µm
Peak power Pa= 500- 3000 W, more preferable 500 - 2000 W
Energy per pulse 0.05 - 1 J, more preferable 0.05 - 0.5 J
Pulse rep rate *v*=10- 3000Hz/Period T=0.0003-0.1s
Na=2-100

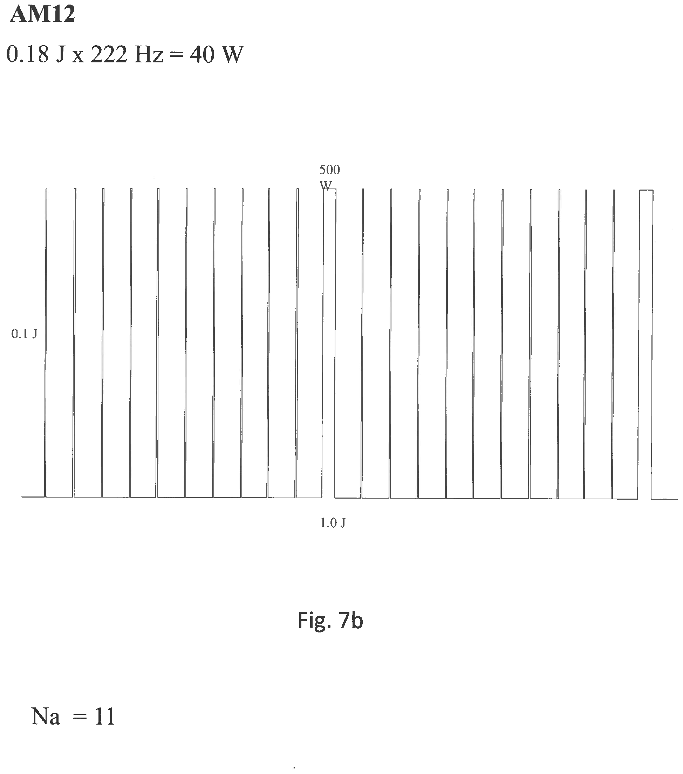

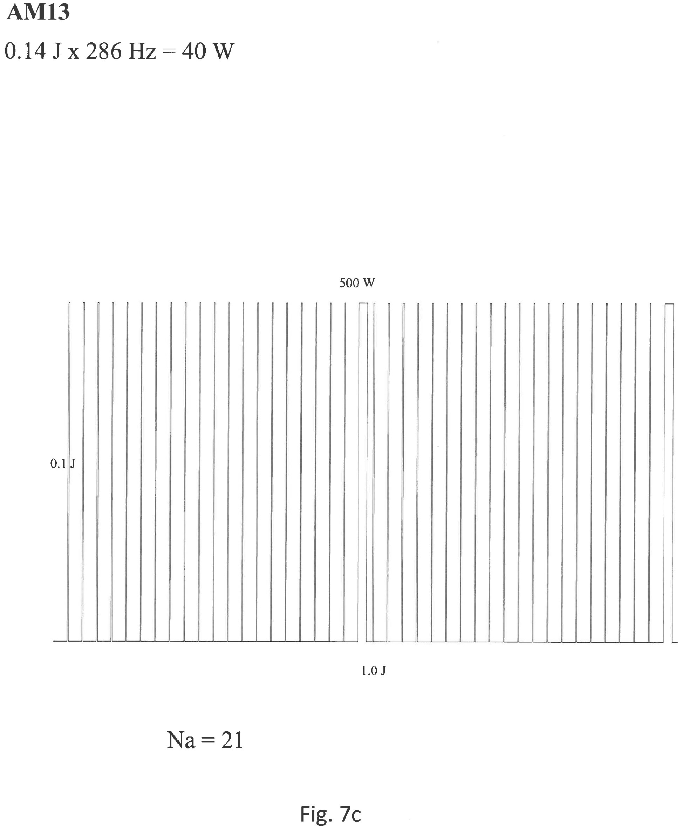

These settings are exemplified by Fig.7 and Table 3.

**Table 3. Examples of AM sequences for non-contact mode of lithotripsy. All experiments were performed with identical average power 40 W in order to match the soft tissue safety profiles for different settings.**

| ***Sequence name*** | ***Figure*** | ***Modulated quantity*** | ***Rep rate, Hz*** | ***Amplitude period, Na*** | ***Dusting speed, mg*/*s*** | ***Normalized dusting speed, au*** |
|---|---|---|---|---|---|---|
| | | | | | | |
| Regular1 with 500W peak power (Reference) | Similar to 4a | None | 400 | | 0.81 ±0.03 | 1 ± 0.08 |
| Regular2 with 1000W peak power | Similar to 4a | None | 40 | | 0.75 ± 0.05 | 0.92 ± 0.07 |
| AM11 | 7a | Energy | 160 | 6 | 1.3 ± 0.04 | 1.6 ± 0.09 |
| AM12 | 7b | Energy | 222 | 11 | 1.2 ± 0.06 | 1.48 ± 0.09 |
| AM13 | 7c | Energy | 286 | 21 | 1.15 ± 0.05 | 1.42 ± 0.06 |

These data suggest that the amplitude modulation can significantly (up to 60%) reduce time required to complete non-contact dusting procedure, while maintaining the average power of the laser constant

### Frequency modulation (FM)

Frequency modulation is a particular case of FM, when the pulse period varies, whereas both pulse energy and peak power remain constant. A typical case of frequency modulation is shown in Fig. 8. Here, the magnitude (*Pp* and *E*) remains constant, while the pulse period varies with the period Np=5. As AFM, FM is characterized by the average pulse period Tav. The group of pulses 801 in the time interval *Tav*Np* forms the periodic pulse group.

Many varieties of the FM pulse groups are possible.

FM can be beneficial for both principal modes of the laser lithotripsy (i.e., contact and non-contact). Summarized below are preferable regimes:

### 1. Contact/Scanning mode

Preferable parameters:
Wavelength 1.81-2.2 µm, more preferable 1.908-1.98 µm
Peak power Pa= 250- 3000 W, more preferable 400 - 1000 W
Energy per pulse 0.02 - 2 J, more preferable 0.05 - 0.5 J
Pulse rep rate *v*=5- 3000Hz/Period T=0.00033-0.2s, more preferable 50 - 1000 Hz, 0.001-0.02s
Np=10-100

### 2. Contact/Fragmentation mode

Preferable parameters:
Wavelength 1.81-2.2 µm, more preferable 1.908-1.98 µm
Peak power Pa= 100 - 3000 W, more preferable 400 - 1000 W
Energy per pulse 0.2 - 20 J, more preferable 0.5 - 5 J
Pulse rep rate *v*=1- 300Hz/Period T=0.0033-1s
Np=10-100
3. Non-contact (popcorning) mode

Preferable parameters:
Wavelength 1.81-2.2, more preferable 1.908-1.98
Peak power Pa= 250- 5000 W, more preferable 250 - 1000 W
Energy per pulse 0.02 - 1J, more preferable 0.05 - 0.5J
Pulse rep rate *v*=10- 1000Hz/Period T=0.001-0.1s
Np=10-100

### Pulse Shape

Laser energy emitting from the fiber end and traveling in a liquid (water) medium in the gap between the fiber end and a stone or tissue surface toward the target stone or tissue will be absorbed, but the absorption may be less than expected, which is attributed to the "Moses effect," where a first component of the emitted energy is absorbed by the liquid and creates a vapor bubble in the liquid medium so that the remaining energy passes through a less-restrictive or absorbing gaseous/vapor medium characterized by a lower optical attenuation. The laser-induced vapor bubble created during the initial pulse functions to "part the water," which enables the subsequent pulse to be more efficiently delivered to the stone. This phenomenon has been proposed to use for increasing efficiency of stone ablation using two pulses: first delivering a short, low-energy pulse that creates a vapor bubble, which is followed by a longer, higher energy treatment pulse (see U.S. Patent No. 5,321,715).

In the present invention, controlling the temporal structure of the laser power is used to minimize the retropulsion effect. The water bubble forming between the stone and the distal fiber end can generate pressure and force to move the stone away from the fiber. This effect can be minimized by decreasing laser power and energy during bubble formation. Collapsing of the bubble between pulses can generate negative pressure and force to the stone and compensate for stone movement due to bubble growth and recoil movement during stone ablation (suction effect). These effect can be controlled by varying the individual pulse shape f(t), pulse energy E, and interval between pulses T.

Laser ablation normally requires the combination of high ablation efficiency and a low retropulsion effect. To compare different temporal laser structures, the laser ablation efficiency η_{abl}, which is defined as the volume of the product of ablation divided by the total laser energy spent to ablate this volume and speed of the stone displacement V due to retropulsion at the very start of laser pulsing can be used. The value of V is determined by the impact of a single pulse for low rep rate or by the impact of a number of pulses during about 0.1s for high rep rate laser system. Specifically, the ratio η_{abl}/Vcan characterize practical (compound) efficiency or speed of treatment.

The pulse shape for a solid state laser configured with flash lamp pumping normally has an irregular spiky structure and can be controlled by current through pumping the flash lamp in a very limited manner. In contrast, diode-pumped fibers and solid-state lasers allow precise control of the pulse shape within a wide range of parameters and increase the speed of treatment.

In the present invention, in addition to the AM and AFM, the temporal structure of the laser emission is controlled through modulating the individual pulse shape f(t) to provide the optimum conditions for stone ablation with the maximum efficiency and reduced retropulsion;

When treating stones in the contact mode, the objective is to increase the efficiency of the stone ablation in order to reduce the total time required for fragmenting the stone. This can be achieved through adjusting the shape of the pulse by applying reduced intensity in the first portion of the pulse to establish a Moses channel with minimal energy losses but at the same time minimize the retropulsion effect of such pulse), followed by applying increased intensity in the second portion of the pulse to maximize the thermal or thermo-mechanical effect on the stone. Water absorption losses of the second portion of the pulse will be greatly reduced due to a Moses channel established by the first portion of the pulse. However, the Moses vaporization bubble or channel, which is growing between the fiber end and the stone, produces pressure and force on the stone and thus creates a retropulsion effect. In the present invention it is proposed to minimize laser pulse peak power and energy to reduce the retropulsion effect. In an experimental setting bubble dynamics were measured at the end of 0.2 mm fiber using a high speed video camera with 120000 frame per second speed. The stone sample's displacement effect of a single pulse exposure was measured. See description of the experimental setup above.

**Table 5. Dimensions of vapor bubbles.**

| ***Energy, J*** | ***Peak power*** | | | | | |
|---|---|---|---|---|---|---|
| | ***100*** | | ***200*** | | ***500*** | |
| | ***Bubble length, mm*** | ***Displacemen t, mm*** | ***Bubble length, mm*** | ***Displacemen t, mm*** | ***Bubble length, mm*** | ***Displacemen t, mm*** |
| 0.02 | 1.1 ± 0.2 | 0.1 ± 0.01 | n/a | n/a | | |
| 0.05 | 1.7 ± 0.3 | 0.3 ± 0.02 | 1.3 ± 0.02 | 0.6 ± 0.02 | | |
| 0.1 | 2.7 ± 0.4 | 0.5 ± 0.02 | 2.4 ± 0.03 | 0.9 ± 0.04 | 4.2 ± 0.1 | 1.1 ± 0.09 |
| 0.2 | 3.3 ± 0.5 | 1 ± 0.03 | 3.4 ± 0.05 | 1.3 ± 0.06 | 5.9 ± 0.2 | 2 ± 0.1 |
| 0.4 | | | | | 6.2 ± 0.4 | 4.3 ± 0.4 |

Table 5 summarizes experimental data for a TFL having a wavelength of 1940 nm and a fiber core of 0.2 mm. The results indicate that the length of the bubble and stone displacement, which is proportional to the bubble pressure, increase with laser pulse peak power and energy. Distances between the fiber end and the stone in a clinical contact setting are in the range between 0 and 1 mm, but during treatment for short period of times it can exceed 2.5 mm. To take advantage of the Moses (vaporized) channel for ablation efficiency but to minimize the retropulsion effect, it is proposed to use laser parameters of the first sub-pulse that create bubbles having a length that is no longer than 2.5 mm with minimal pressure. Based on a measured peak power data of the first laser sub-pulse, which creates a Moses (vapor) channel, the peak power should be in the range 50 - 500 W, preferably 100 -300 W and the energy per pulse should be 0.02 to 0.15 J, preferably 0.05 - 0.1 J. The interval between the first and the second sub-pulses for efficient ablation should be defined based on the following criteria: 1) The second sub-pulse should be started after the vapor channel front reaches the stone, i.e., when the bubble has grown to 2.5 mm, preferably 1 mm; 2) The pressure on the bubble has dropped or has become negative in order to produce a stone suction effect.

**Table 6. Growth times of vapor bubbles.**

| ***Energy,* J** | ***Peak power*** | | | | | |
|---|---|---|---|---|---|---|
| | ***100*** | | ***200*** | | ***500*** | |
| | ***Time of growth to 1 mm, µs*** | ***Time to growth to 2.5 mm, µs*** | ***Time of growth to 1 mm, µs*** | ***Time to growth to 2.5 mm, µs*** | ***Time of growth to 1 mm, µs*** | ***Time to growth to 3 mm, µs*** |
| 0.02 | 100 ± 8 | | n/a | n/a | | |
| 0.05 | 200 ± 12 | | 100 ± 8 | | | |
| 0.1 | 170 ± 14 | | 80 ± 6 | | 30 ± 8 | 210 ± 18 |
| 0.2 | 150 ± 8 | 900 ± 24 | 50 ± 5 | 500 ± 24 | 40 ± 10 | 190 ± 16 |
| 0.4 | | | | | 20 ± 5 | 170 ± 14 |

Table 6 shows the time duration for bubble growth to 1 mm and 3 mm as a function of laser peak power and energy from the proposed range. Hence, the interval between the sub-pulses should be in the range 50-900 µs, preferably 100-500 µs. The energy of the second sub-pulse should be in the range from 0.1 to 10 J. Such a pulse shape is illustrated by Fig. 9.

In other embodiments of the present invention, we propose the pulse shape with continuously delivered power during the pulse. This shape is the most effective for fragmentation mode when operator uses drilling technique while providing close contact between laser fiber end and stone during all treatment cycle. In this case, water layer between the fiber end and stone can be very minimal (below 0.5 mm) or not present at all. Laser that is currently used for lithotripsy has uniform rectangular or flat-top pulse, which is typical for diode pumped fiber and solid state lasers 202 (Fig 2). Flash pumped solid state lasers such as Ho:YAG have asymmetrical shape with higher power in the beginning of pulse and slow relaxation of power on the back tail 203 or 204 (Fig. 2). These shapes of pulse are not optimal for stone cracking during drilling. In order to increase efficiency of ablation during drilling and fragmentation, in the present invention we propose using pulse with two portions, where the first portion is used for removing residual water between the fiber and the stone and ablation of stone and preheating of stone around ablation crater (Fig.10). Preheating by the first portion of the pulse with lower power will result in increasing of thermal stress around laser crater and increasing the coefficient of absorption of stone matrix due to heating above 100 -250° C. The second portion of the pulse with higher power will be more effectively absorbed by stone material and will produce more efficient mechanical damage due to better absorption than for the first pulse portion and higher peak power of second portion and initial mechanical stress in stone around laser crater. As a result, the probability of stone cracking into large parts will be increased. At the same time, retropulsion effect of such pulsing will be decreased due to more efficient transformation of laser energy into cracking rather than to ablation of small particles with high recoil moment. This is illustrated by Fig. 10, where *τ₁* is the duration of the first portion of the pulse and *τ₂* is the duration of the second portion of the pulse. Power profile of the first portion of the pulse f₁(t) can be constant on the level Pₘᵢₙ or a monotonic function, such as linear, exponential, polynomic functions increasing from Pₘᵢₙ to Pₘₐₓ, where Pₘₐₓ is peak power of the second portion of the pulse. Other temporal dependencies of the intensity are also possible and will be obvious to those skilled in the art. Duration of the first portion of the pulse can be determined from considering the minimal energy required for establishing Moses channel and produce ablation of the stone with substantial heating of stone matrix around laser crater to increase stone matrix absorption and to enhance stone macro cracking effect. In alternative embodiments, duration of the first portion can be determined in real time, using a feedback mechanism. Feedback mechanism will signal establishment of the Moses channel and/or stone crater temperature, and can be based on optical, acoustical, or other technologies. For example, stone temperature can be detected by means of measuring the thermal radiation emitted by the stone through the same fiber that is used for laser power delivery. For TFL with wavelength 1.94 µm, we have found experimentally that the laser power of the first portion of the pulse should be in the range Pₘᵢₙ=50 - 200W. the pulse duration *τ₁* should be in the range 0.1 to10 ms, with energy of first portion 10-70% of total energy of pulse, whereas the power of the second portion of the pulse should be in the range 400 to 20000 W and its duration in the range 0.5 to 20 ms.

Examples of pulse shapes optimized for stone drilling and fragmentation are given in Fig.11 and Table 7.

**Table 7. Pulse shapes preferable for stone drilling and fragmentation. All experiments were performed with identical average power 9 W in order to match the soft tissue safety profiles for different settings.**

| ***Pulse shape name*** | ***Figure No.*** | ***Rep rate, Hz*** | ***Time to cracking, sec*** | ***Retropulsion speed, mm*/ *s*** | ***Normalized time fragmentation*/*retropuls ion ratio*** |
|---|---|---|---|---|---|
| | | | | | |
| Regular1 (Reference) | n/a | 6 | 2.7 ± 0.4 | 17.7 ± 2.0 | 1 ± 0.1 |
| Regular2 | n/a | 6 | 1.3 ± 0.3 | 37 ± 0.5 | 1 ± 0.1 |
| | | | | | |
| SP1 | 11a | 6 | 1.1 ± 0.2 | 25.7 ± 0.1 | 1.7 ± 0.2 |
| SP2 | 11b | 6 | 1.2 ± 0.2 | 17.4 ± 1.8 | 2.3 ± 0.3 |
| SP3 | 11c | 3 | 1.0 ± 0.2 | 21.1 ± 0.3 | 2.2 ± 0.02 |

Table 7 shows that increasing peak power in regular regimes leads to decreasing cracking time, but also increases the retropulsion effect, so that the resulting regimen quality remains nearly the same. In contrast, shaping of pulse proposed in the present invention decreases both cracking time and retropulsion effect, thus leading to the desired increase in the regimen quality.

The foregoing description and examples have been set forth merely to illustrate the disclosure and are not intended to be limiting. Accordingly, disclosure should be construed broadly to include all variation within the scope of the appended claims.

Especially this invention refers to the following aspects:
Aspect 1. A laser system for treating calculi in a human or animal body, comprising:
   a laser emitting a sequence of laser pulses, the laser being operable in an amplitude-modulation regime (AMR) in which the laser pulses are emitted:
   at a constant pulse frequency (PF), and
   with a periodically varying peak power or pulse energy or peak power and pulse energy with an amplitude modulation period Na equal to the number of pulses in an amplitude periodic group of pulses.
Aspect 2. A laser system for treating calculi in a human or animal body, comprising:
   a laser emitting a sequence of laser pulses, the laser being operable in an amplitude-frequency modulation (AFMR) regime in which the laser pulses are emitted with:
   a periodically varied at least one of pulse peak power and pulse energy with a modulation period Na equal to the number of pulses in an amplitude periodic group of pulses, and
   a periodically varied PF with a frequency modulation period Np equal to the number of pulses in a frequency periodic group of pulses.
Aspect 3. The laser system of aspect 1 or 2, wherein the modulation period Na of at least one of peak power and pulse energy ranges from 2 to 1000 laser pulses, preferably from 2 to 100 laser pulses, most preferably from 2 to 10 laser pulses.
Aspect 4. The laser system of aspect 2, wherein the modulation period Np of the PF varies from 2 to 1000 laser pulses, preferably from 2 to 100 laser pulses, most preferably from 2 to 10 laser pulses.
Aspect 5. The laser system of aspect 1 or 2 wherein the laser is selected from a diode-pumped solid state laser, a diode-pumped fiber laser, a flash lamp-pumped solid state laser, or a direct diode laser.
Aspect 6. The laser system of aspect 1 or 2, wherein the laser operates in a wavelength range of 1.85 to 2.2 µm and preferably in a 1.908 to 1.96 µm.
Aspect 7. The laser system of aspect 6, wherein the laser is one of a Tm:YAG, Tm:YLF, Tm:YAP, Tm: LuAG, Tm:LuLF, Tm:LuAP, Tm fiber, or Ho:YAG laser.
Aspect 8. The laser system of aspect 1 or 2, wherein the laser operates in a free running mode outputting the sequence of laser pulses with the PF ranging between 2 and 5000 Hz, each laser pulse being characterized with:
   a laser pulse energy in a 0.01 J - 10 J range,
   a laser pulse peak power in a 100 - 20000 W range, preferably 250 - 3000 W range, and
   a laser pulse duration in a 25 µs - 20 ms range, with a 50 µs - 10 ms range being preferable.
Aspect 9. The laser system of aspect 1 or 2 further comprising
   a controller outputting a control signal containing information on the desired peak power or pulse energy in the AMR or desired peak power or pulse energy, and desired PF in the AFMR;
   a driver coupled to the controller and operative to output a sequence of electrical current pulses which are periodically modulated and coupled into an input of a pump, which energizes the laser.
Aspect 10. The laser system of aspect 9, wherein the laser driver further comprises an energy storage implement operatively coupled to the controller.
Aspect 11. The laser system of aspect 9, further comprising an acoustic-optical modulator (AOM), an electro-optical modulator (EOM) or a passive modulator coupled to the controller and the laser and operating in a Q-mode with modulated quality of the resonator, outputting the laser pulses each which being characterized by
   an energy varying between 0.1 and 10 mJ,
   a peak power ranging between 200 and 1000000W, and
   a PF ranging between 500 and 500000 Hz.
Aspect 12. The laser system of aspect 1, wherein the laser operating in the AMR in a scanning surgical procedure outputs the sequence of optical pulses at:
   the PF = 5- 3000 Hz, more preferable a 50 -1000 Hz range,
   the amplitude modulation period Na ranging between 2 and 10 optical pulses,
   a 1.81-2.2 µm wavelength range with a 1.908-1.98 µm being preferable,
   the optical pulses each having a 250 to 5000 W peak power range with a 400 to 1000 W range being preferable,
   and the energy per pulse in the range 0.01 to 2 J, preferably 0.05 to 0.5 J
Aspect 13. The laser system of aspect 2, wherein the laser operating in the AFMR regime during a fragmentation surgical procedure outputs the sequence of optical pulses at
   the PF varying between 1 and 5000 Hz,
   the amplitudes modulation period Na ranging between 2 and 10 optical pulses, and
   the frequency modulation period Np ranging between 1 and 100 optical pulses:
      the optical pulses each being output at
      a 1.81-2.2 µm wavelength range with a 1.908-1.98 µm being preferable,
      a peak power range between 100 and 20000 W, with a 250 - 3000 W being preferable,
      an energy per pulse varying between 0.2 - 20 J, and preferably between 0.5- 10 J.
Aspect 14. The laser system of aspect 2, wherein the laser operating in the AFMR regime in a non-contact surgical procedure outputs the sequence of optical pulses at:
   the PF varying between 10 and 3000Hz,
   the amplitudes modulation period Na ranging between 2 and 100 optical pulses, and
   the frequency modulation period Np ranging between 1 and 100 optical pulses, the optical pulses each being output
   at a 1.81-2.2 µm wavelength range with a 1.908-1.98 µm being preferable,
   with a peak power in a 250 and 3000 W range, with a 250 - 1000 W being preferable,
   with an energy per pulse varying between 0.02 - 1 J and preferably in a 0.05-0.5 J range.
Aspect 15. The laser system of aspect 1 or 2 further comprising a fiber guiding the laser pulses to the calculi.
Aspect 16. A laser system for treating calculi in a human or animal body, comprising:
   a controller outputting a control signal containing information on a desired laser pulse shape;
   a laser emitting a sequence of laser pulses and operatively coupled to the controller so that each laser pulse has a desired laser pulse shape which is formed with:
      a first and second sub-pulses spaced temporarily from one another;
      an energy of the first sub-pulse varying in a range of 0.02 to 0.15 J (preferably 0.05 to 0.1 J);
      a peak power of the first sub-pulse varying in a range of 50 to 500 W (preferably 100 to 300 W);
      an interval between the first and the second sub-pulses varying in a range 50 to 900 us with a 100 to 500 us range being preferable;
      an energy of the second sub-pulse exceeding the energy of the first sub-pulse and varying in a range of 0.1 to 10 J;
      a peak power of the second sub-pulse exceeding the peak power of the first sub-pulse and varying in a range of 300 to 20000 W.
Aspect 17. A laser system for treating calculi in a human or animal body, comprising:
   a controller outputting a control signal containing information on a desired laser pulse shape; and
   a laser emitting a sequence of laser pulses and operatively coupled to the controller so that each laser pulse is formed with the desired laser pulse shape including:
      an initial and subsequent sections, with the subsequent section having a higher power level than the initial section,
      the power of the initial section being monotonically increased from a minimal power level to the power level of the subsequent section;
      the minimal power level of the initial pulse section varying between 0 and 200 W,
      the duration of the initial section varying in a 0.1 to 10 ms range;
      the energy of the initial pulse section being 10-70% of a total energy of pulse;
      the power of the subsequent section varying between 400 and 20000 W; and
      the duration of the subsequent section varying in a 0.5 to 20 ms range.
Aspect 18. The laser system of aspect 17, wherein the power of the initial section increases in accordance with one of linear, polynomial, and exponential functions.
Aspect 19. The laser system of aspect 16 or 17, wherein the laser is selected from the following group: a diode-pumped solid state laser, a diode-pumped fiber laser, a flashlamp-pumped solid state laser, or a direct diode laser.
Aspect 20. The laser system of aspect 18, wherein the laser is one of a Tm:YAG, Tm:YLF, Tm:YAP, Tm:LuAG, Tm:LuLF, Tm:LuAP Tm fiber, or Ho:YAG laser.
Aspect 21. The laser system of aspect 16 or 17, wherein the laser operates in a wavelength range of 1.85 to 2.2 µm and preferably in a 1.908 to 1.96 µm.
Aspect 22. The laser system of aspect 16 or 17 further comprising a fiber guiding the laser pulses to the calculi.
Aspect 23. A method for treating calculi in a human or animal body, comprising:
   emitting a sequence of laser pulses from a laser at a constant pulse frequency (PF), and with a periodically varying peak power or pulse energy or peak power and pulse energy with an amplitude modulation period Na ≥ 2, equal to the number of pulses in the periodic amplitude group of pulses.
Aspect 24. A method for treating calculi in a human or animal body, comprising:
   emitting a sequence of laser pulses by:
   periodically varying at least one of pulse peak power or pulse energy or pulse peak power and energy with a modulation period Na equal to the number of pulses in an amplitude periodic group of pulses, and
   periodically varying PRF with a frequency modulation period Np equal to the number of pulses in a frequency periodic group of pulses.
Aspect 25. The method of aspect 23 or 24, wherein the modulation period Na of at least one of peak power and pulse energy ranges from 2 to 1000 laser pulses, preferably from 2 to 100 laser pulses, most preferably from 2 to 10 laser pulses.
Aspect 26. The method of aspect 23 or 24, wherein the modulation period Np of the PRF varies from 2 to 1000 laser pulses, preferably from 2 to 100 laser pulses, most preferably from 2 to 10 laser pulses.
Aspect 27. The method of aspect 23 or 24 wherein the laser is selected from a diode-pumped solid state laser, a diode-pumped fiber laser, a flashlamp-pumped solid state laser, or a direct diode laser.
Aspect 28. The method of aspect 23 or 24, wherein the laser operates in a wavelength range of 1.85 to 2.2 µm and preferably in a 1.91 to 1.96 µm wavelength range.
Aspect 29. The method of aspect 23 further comprising delivering the sequence of laser pulses in a scanning surgical procedure at
   a pulse frequency PF ranging varying from 5 to 3000Hz, and preferably from a 50 -1000 Hz range,
   the amplitude modulation period Na ranging between 2 and 10 optical pulses,
   a 1.81-2.2 µm wavelength range with a 1.908-1.98 µm being preferable,
   wherein the optical pulses each have a 250 to 5000 W peak power range with a 400 - 1000 W range being preferable,
   and energy per pulse in the range 0.01 to 2 J, preferably 0.05 to 0.5 J .
Aspect 30. The method of aspect 23 further comprising delivering the sequence of laser pulses in a fragmentation surgical mode at:
   the PF varying between 1 and 300Hz,
   the amplitude modulation period Na ranging between 2 and 10 laser pulses,
   at a 1.81-2.2 µm wavelength range with a 1.908-1.98 µm being preferable,
   wherein the optical pulses each are characterized by a 100 and 20000 W peak power range, with a 250 - 1000 W range being preferable, and an energy per pulse varying between 0.2 - 20 J, with a 0.5-5 J preferred range.
Aspect 31. The laser system of aspect 24 further comprising delivering the sequence of laser pulses in a fragmentation surgical procedure at:
   the PF varying between 1 and 3000Hz,
   the amplitudes modulation period Na ranging between 2 and 10 optical pulses, and
   the frequency modulation period Np ranging between 1 and 100 optical pulses:
      the optical pulses each being output at
      a 1.81-2.2 µm wavelength range with a 1.908-1.98 µm being preferable,
      a peak power range between 100 and 20000 W, with a 250 - 3000 W being preferable,
      an energy per pulse varying between 0.2 - 20 J, and preferably between 0.5- 10 J.
Aspect 32. The laser system of aspect 24 further comprising delivering the sequence of laser pulses in a non-contact surgical procedure at:
   the PF ν varying between 10 and 1000Hz,
   the amplitudes modulation period Na ranging between 2 and 100 optical pulses, and
   the frequency modulation period Np ranging between 1 and 100 optical pulses,
   the optical pulses each being output
   at a 1.81-2.2 µm wavelength range with a 1.908-1.98 µm being preferable,
   with a peak power in a 250 and 5000 W range, with a 250 - 1000 W being preferable,
   with an energy per pulse varying between 0.05 - 1 J and preferably in a 0.05-0.5 J range.
Aspect 33. A method for treating calculi in a human or animal body, comprising:
   outputting a control signal containing information on a desired laser pulse shape;
   emitting a sequence of laser pulses each with the desired laser shape which is formed with:
      a first and second sub-pulses spaced temporarily from one another;
      an energy of the first sub-pulse varying in a range of 0.02 to 0.15 J (preferably 0.05 to 0.1 J);
      a peak power of the first sub-pulse varying in a range of 50 to 500 W (preferably 100 to 300 W);
      an interval between the first and the second sub-pulses varying in a range 50 to 900 us with a 100 to 500 us range being preferable;
      an energy of the second sub-pulse exceeding the energy of the first sub-pulse and varying in a range of 0.1 to 10 J;
      a peak power of the second sub-pulse exceeding the peak power of the first sub-pulse and varying in a range of 300 to 20000 W.
Aspect 34. A method for treating calculi in a human or animal body, comprising:
   outputting a control signal containing information on a desired laser pulse shape; and
   emitting a sequence of laser pulses each with the desired laser pulse shape including:
      an initial and subsequent sections, with the subsequent session having a higher power level than the initial section,
      the power of the initial section being monotonically increased from a minimal power level to the power level of the subsequent session;
      the minimal power level of the initial pulse section varying between 0 and 200 W,
      the duration of the initial section varying in a 0.1 to 10 ms range;
      the energy of the initial pulse section being 10-70% of a total energy of pulse;
      the power of the subsequent section varying between 400 and 20000 W; and
      the duration of the subsequent section varying in a 0.5 to 20 ms range.
Aspect 35. The method of aspect 34 further comprising increasing the power of the initial section in accordance with one of linear, polynomial, and exponential functions.
Aspect 36. The method of aspect 33 or 34, wherein the laser is selected from the following group: a diode-pumped solid state laser, a diode-pumped fiber laser, a flash lamp-pumped solid state laser, or a direct diode laser.
Aspect 37. The method of aspect 36, wherein the laser is one of a Tm:YAG, Tm:YLF, Tm:YAP, Tm:LuAG, Tm:LuLF, Tm:LuAP, Tm fiber, or Ho:YAG laser.
Aspect 38. The method of aspect 33 or 34, wherein the laser operates in a wavelength range of 1.85 to 2.2 µm and preferably in a 1.908 to 1.96 µm wavelength range.
Aspect 39. The method of aspect 33 or 34 further guiding the sequence of laser pulses incident on a calculus in a human or animal body through a delivery fiber.

## Claims

1. A laser system for treating calculi in a human or animal body, comprising:
a diode-pumped laser configured to emit radiation in a wavelength range of 1.85 µm to 2.2 µm; and
a driver configured to provide modulated current to one or more pump diodes of the diode-pumped laser and coupled to a controller configured to output a control signal containing information on a desired laser pulse shape such that the diode-pumped laser emits a sequence of laser pulses each having the desired laser pulse shape, wherein:
the desired laser pulse shape is formed with an initial pulse section and a subsequent pulse section,
the subsequent pulse section has a higher power than a power of the initial pulse section, and the power of the initial pulse section is monotonically increased from a minimal power level to the power of the subsequent pulse section,
the minimal power level of the initial pulse section varying in a range of 0 W to 200 W,
a duration of the initial pulse section varying in a range of 0.1 ms to 10 ms,
an energy of the initial pulse section varying in a range of 10 % to 70 % of a total energy of the laser pulse,
the power of the subsequent pulse section varying in a range of 400 W to 20 kW, and
a duration of the subsequent pulse section varying in a range of 0.5 ms to 20 ms.

2. The laser system of claim 1, wherein the diode-pumped laser is a thulium fiber laser or a Tm:YAG laser.

3. The laser system of claim 1, wherein the power of the initial pulse section increases in accordance with one of linear, polynomial, and exponential functions.

4. The laser system of claim 1, further comprising a fiber configured to guide the laser pulses to the calculi.

5. The laser system of claim 4, wherein the initial pulse section removes residual water between the fiber and the calculi.

6. The laser system of claim 5, wherein the subsequent pulse section produces mechanical damage in the calculi.

7. The laser system of claim 1, wherein the duration of the initial pulse section is determined in real time using a feedback mechanism.

8. The laser system of claim 7, further comprising a fiber configured to guide the laser pulses to the calculi, wherein the feedback mechanism signals a temperature of the calculi, the temperature being detected by measuring thermal radiation emitted by the calculi through the fiber.

9. The laser system of claim 1, wherein the diode-pumped laser is a thulium fiber laser emitting radiation at a wavelength of 1.94 µm, and the minimal power level of the initial pulse section varies in a range of 50 W to 200 W.
